# EUROPÄISCHE PATENTSCHRIFT

(11) **EP 1 501 566 B1**
(45) Veröffentlichungstag und Bekanntmachung des Hinweises auf die Patenterteilung: **13.08.2008**
(21) Anmeldenummer: 03749833.4
(22) Anmeldetag: 15.04.2003
(51) Int. Cl.: A61L 31/16, A61L 33/08, A61K 31/727

(54) **HEMOKOMPATIBEL BESCHICHTETE MEDIZINPRODUKTE, DEREN HERSTELLUNG UND VERWENDUNG**
MEDICAL PRODUCTS COMPRISING A HAEMOCOMPATIBLE COATING, PRODUCTION AND USE THEREOF
PRODUITS MEDICAUX POURVUS D'UN REVETEMENT HEMOCOMPATIBLE, LEUR PRODUCTION ET LEUR UTILISATION

(30) Priorität: 09.05.2002 US 378676 P; 10.05.2002 DE 10221055
(43) Veröffentlichungstag der Anmeldung: 02.02.2005
(73) Patentinhaber: Hemoteq AG, 52146 Würselen (DE)
(72) Erfinder: HORRES, Roland, 52223 Stolberg (DE); LINSSEN, Marita Katharina, 52066 Aachen (DE); HOFFMANN, Michael, 52249 Eschweiler (DE); FAUST, Volker, 52080 Aachen (DE); HOFFMANN, Erika, 52249 Eschweiler (DE); DI BIASE, Donato, 52080 Aachen (DE)
(74) Vertreter: Arth, Hans-Lothar
(86) Internationale Anmeldenummer: PCT/DE2003/001254
(87) Internationale Veröffentlichungsnummer: WO 2003/094991

(56) Entgegenhaltungen:
- WO-A-00/21584
- WO-A-99/26983
- WO-A-99/27976
- US-A1- 2001 032 014

## Beschreibung

Die Erfindung betrifft die Verwendung von Polysacchariden, enthaltend den Zuckerbaustein N-Acylglucosamin für die Herstellung hemokompatibler Oberflächen von Medizinprodukten, Verfahren zur hemokompatiblen Beschichtung von Oberflächen mit diesen Polysacchariden sowie Medizinprodukte mit derartigen hemokompatiblen Oberflächen.

Im menschlichen Körper kommt das Blut nur im Falle einer Verletzung mit anderen Oberflächen in Kontakt als der Innenseite von natürlichen Blutgefäßen. Daher wird das Blutgerinnungssystem immer dann aktiviert, wenn Blut mit fremden Oberflächen in Kontakt kommt, um die Blutung zu stillen und einen lebensbedrohlichen Blutverlust zu verhindern. Da ein Implantat ebenfalls eine fremde Oberfläche darstellt, werden alle Patienten, die ein Implantat erhalten, das dauerhaft mit Blut in Kontakt steht, für die Dauer des Blutkontaktes mit Medikamenten, sogenannten Antikoagulantien, welche die Blutgerinnung unterdrücken, behandelt, wobei zum Teil erhebliche Nebenwirkungen in Kauf genommen werden müssen.

Das beschriebene Thromboserisiko tritt auch als einer der Risikofaktoren beim Einsatz von Gefäßstützen, sogenannten Stents, in blutführenden Gefäßen auf. Der Stent dient zur Aufdehnung der Gefäßwände beim Auftreten von Gefäßverengungen und -verschlüssen z.B durch artheriosklerotische Veränderungen speziell der Herzkranzgefäße. Er fixiert Kalkfragmente in den Gefäßen und verbessert die Fließeigenschaften des Blutes im Gefäß, indem er die Oberfläche des Gefäßinnenraums glättet. Außerdem bewirkt ein Stent einen Widerstand gegen elastische Rückstellkräfte des aufgedehnten Gefäßabschnittes. Das verwendete Material ist in der Regel medizinischer Edelstahl.

Die Stentthrombose tritt in weniger als einem Prozent der Fälle noch im Herzkatheterlabor als frühe Thrombose oder in zwei bis fünf Prozent der Fälle während des Krankenhausaufenthaltes auf. Gefäßverletzungen durch den Eingriff werden in etwa fünf Prozent der Fälle aufgrund der arteriellen Schleusen verursacht und es besteht auch die Möglichkeit durch Ausbuchtung von Gefäßen Pseudo-Aneurysmen zu verursachen. Die kontinuierliche Gabe von Heparin als Antikoagulanz erhöht zusätzlich das Blutungsrisiko.

Eine weitere sehr häufig auftretende Komplikation ist die Restenose, der Wiederverschluß des Gefäßes. Obwohl Stents das Risiko eines erneuten Gefäßverschlusses verkleinern, sind sie bisher nicht in der Lage, Restenosen vollständig zu verhindern. Die Wiederverengungsrate (Restenose) nach einer Stentimplantation mit bis zu 30 % ist eine der Hauptursachen für den erneuten Aufenthalt von Patienten in der Klinik.

Eine genaue begriffliche Beschreibung der Restenose ist in der Fachliteratur nicht aufzufinden. Die am häufigsten verwendete morphologische Definition der Restenose ist diejenige, die nach erfolgreicher PTA (perkutane transluminale Angioplastie) die Restenose als eine Reduktion des Gefäßdurchmessers auf weniger als 50% des normalen festlegt. Hierbei handelt es sich um einen empirisch festgelegten Wert, dessen hämodynamische Bedeutung und Beziehung zur klinischen Symptomatik einer soliden wissenschaftlichen Basis entbehrt. In der Praxis wird häufig die klinische Verschlechterung eines Patienten als Zeichen einer Restenose des vormals behandelten Gefäßabschnitts angesehen.

Die während der Implantation des Stents verursachten Gefäßverletzungen rufen Entzündungsreaktionen hervor, die für den Heilungsprozeß in den ersten sieben Tagen eine entscheidende Rolle spielen. Die hierbei ablaufenden Prozesse sind unter anderem mit der Ausschüttung von Wachstumsfaktoren verbunden, womit eine verstärkte Proliferation der glatten Muskelzellen eingeleitet wird und damit schon kurzfristig zu einer Restenose, einem erneuten Verschluß des Gefäßes aufgrund unkontrollierten Wachstums führen. Auch noch nach einigen Wochen, wenn der Stent in das Gewebe des Blutgefäßes eingewachsen und vollständig von glatten Muskelzellen umhüllt wird, können Vernarbungen zu stark ausgeprägt sein (Neointimahyperplasie) und dazu führen, daß nicht nur die Stentoberfläche bedeckt, sondern der ganze Innenraum des Stents verschlossen wird.

Man hat vergeblich versucht, das Problem der Restenose durch die Beschichtung der Stents mit Heparin zu lösen (J. Whörle et al, European Heart Journal (2001) 22 1808-1816). Heparin adressiert als Antikoagulanz jedoch nur die erstgenannte Ursache und kann darüber hinaus nur in Lösung seine volle Wirkung entfalten. Dieses erste Problem läßt sich mittlerweile medikamentös durch die Gabe von Antikoagulantien fast vollständig vermeiden. Das weitere Problem versucht man zur Zeit zu lösen, indem man das Wachstum der glatten Muskelzellen lokal am Stent hemmt. Das wird z.B. mit radioaktiven Stents oder mit Stents versucht, welche pharmazeutische Wirkstoffe enthalten.

Somit besteht ein Bedarf an nicht-thrombogenen, hemokompatiblen Werkstoffen, welche nicht als Fremdoberfläche erkannt werden und bei Blutkontakt nicht das Gerinnungssystem auslösen und zur Koagulation des Blutes führen, womit ein wichtiger Faktor für die restenosefördernden Prozesse ausfällt. Unterstützung sol! durch die Zugabe von Wirkstoffen gewährleistet sein, die Enzündungsreaktionen zurückdrängen sollen oder die die Wundheilung begleitende Zellteilung kontrollieren soll.

Einen hemokompatiblen Werkstoff sowie ein Verfahren zu dessen Herstellung offenbart die internationale Patentanmeldung PCT/EP98/07668 (Veröffentlichungsnummer WO 99/27976 A), worin N-desulfatierte Heparine und nicht thrombogene Polymere enthaltend ein Basispolymer beschrieben werden, an welches diese Heparine kovalent gebunden sind. Als Basispolymer werden bevorzugt Polyvinylchlorid oder Silicon eingesetzt.

Einen anderen Werkstoff zur Verhinderung von arterieller Thrombose offenbart WO 99/26983 A. Darin werden heparinähnliche Verbindungen umfassend Heparine oder heparinähnliche Glykosaminoglykane als solche oder als Proteoglykane oder gebunden an ein globuläres Kernmolekül beschrieben. Die Antithrombose-Eigenschaften ergeben sich aus der hohen Kopplungsdichte von negativ geladenem Heparin oder den heparinähnlichen Glykosaminoglykanen, welche direkt oder über einen Spacer oder Linker an die globulären Kernmoleküle gebunden werden.

Die Anstrengungen auf diesem Gebiet, einen Stent darzustellen, der die Restenose derart reduziert oder sogar eliminiert, sind sehr groß. Dabei werden verschiedene Möglichkeiten der Ausführung in unzähligen Studien untersucht.
Die häufigste Ausführungsform besteht aus einem Stent, der mit einer geeigneten Matrix, einem üblicherweise biostabilen Polymeren, beschichtet wird. Die Matrix enthält einen antiproliferativen oder antiphlogistischen Wirkstoff, der in zeitlicher Folge kontrolliert abgegeben wird und die Entzündungsreaktionen und die überschießende Zellteilung unterdrücken soll.

So offenbart US-A-5 891 108 beispielsweise einen hohl ausgeformten Stent, welcher in seinem Inneren pharmazeutische Wirkstoffe enthalten kann, welche durch eine Vielzahl von Öffnungen im Stent freigesetzt werden. EP-A-1 127 582 beschreibt hingegen einen Stent, der auf seiner Oberfläche Einbuchtungen von 0,1 - 1 mm Tiefe und 7 - 15 mm Länge aufweist, welche zur Aufnahme eines Wirkstoffes geeignet sind. Diese Wirkstoffreservoire setzen, vergleichbar der Öffnungen in dem hohlen Stent, den enthaltenen pharmazeutischen Wirkstoff punktuell in hoher Konzentration und über einen relativ langen Zeitraum frei, was aber dazu führt, daß die glatten Muskelzellen nicht mehr oder nur sehr verzögert in der Lage sind, den Stent zu umhüllen. Daher ist der Stent viel länger dem Blut ausgesetzt, was wieder vermehrt zu Gefäßverschlüssen durch Thrombosen führt (Liistro F., Colombo A., Late acute thrombosis after Paclitaxel eluting stent implantation. Heart (2001) 86 262-4).

Ein Lösungsversuch für dieses Problem stellt die Phosphorylcholinbeschichtung von Biocompatibles (WO 0101957) dar, indem hier Phosphorylcholin, eine Zellmembrankomponente der Erythrocyten, als Bestandteil der aufgebrachten nicht bioabbaubaren Polymerschicht auf dem Stent eine nichtthrombogene Oberfläche erzeugen soll. Dabei wird der Wirkstoff abhängig vom Molekulargewicht von dieser polymerhaltigen Phosphorylcholinschicht absorbiert oder auf der Oberfläche adsorbiert.

Aufgabe der vorliegenden Erfindung ist es, hemokompatibel beschichtete Medizinprodukte sowie Verfahren zur hemokompatiblen Beschichtung zur Verfügung zu stellen.

Insbesondere ist es die Aufgabe der vorliegenden Erfindung Stents bereitzustellen, welche ein kontinuierliches kontrolliertes Einwachsen des Stents erlauben - einerseits durch Unterdrückung der zellulären Reaktionen in den ersten Tagen und Wochen nach Implantation mit Hilfe der ausgewählten Wirkstoffe und Wirkstoffkombinationen und andererseits durch die Bereitstellung einer athrombogenen bzw. inerten bzw. biokompatiblen Oberfläche, die gewährleistet, dass mit Abklingen des Wirkstoffeinflusses keine Reaktionen auf die bestehende Fremdoberfläche mehr einsetzen, die langzeitlich ebenfalls zu Komplikationen führen können.

Die Bemühungen zur Darstellung einer möglichst perfekten Simulation der nativen athrombogenen Verhältnisse auf der dem Blut zugewandten Seite eines Blutgefäßes sind groß. EP-B-0 333 730 beschreibt ein Verfahren zur Herstellung hemokompatibler Substrate durch Einarbeitung, Adhäsion und/oder Modifizierung und Verankerung von nicht-thrombogenem Endothelzelloberflächen-Polysaccharid (HS I). Die Immobilisierung dieses spezifischen Endothelzelloberflächenproteoheparansulfats HS I auf biologischen oder künstlichen Oberflächen bewirkt, daß derartig beschichtete Oberflächen blutverträglich werden und für den dauerhaften Blutkontakt geeignet sind. Nachteilig ist hingegen, daß dieses Verfahren für die Gewinnung von HS I die Kultivierung von Endothelzellen voraussetzt, so daß die wirtschaftliche Verwertbarkeit dieses Verfahrens stark eingeschränkt ist, da die Kultivierung von Endothelzellen zeitaufwendig ist und größere Mengen an kultivierten Endothelzellen nur mit immensem Kostenaufwand erhältlich sind.

Die vorliegende Erfindung löst die Aufgabe durch die Bereitstellung von Medizinprodukten, welche eine Oberflächenbeschichtung aus bestimmten Polysacchariden und Paclitaxel aufweisen. Anstelle von oder zusammen mit Pclitaxel können bestimmte andere antiphlogistische als auch antiinflammatorische Wirkstoffe bzw. Wirkstoffkombination aus Simvastatin, Tialin (2-Methylthiazolidin-2,4-dicarbonsäure), Tialin-Natrium (Natriumsalz von Tialin), Macrocyclisches Suboxid (MCS) und seinen Derivate, Tyrphostine, D24851, Thymosin a-1, Interleukin-1β-Inhibitoren, aktiviertes Protein C (aPC), MSH, Fumarsäure und Fumarsäureester, PETN (Pentaerythrittetranitrat), PI88, Dermicidin, Baccatin und seine Derivate, Docetaxel und weitere Derivate des Paclitaxel, Tacrolimus, Pimecrolimus, Trapidil, α- und β-Estradiol, Sirolimus, Colchicin, und Melanocyte-stimulating hormon (α-MSH) verwendet werden. Verfahren zur Herstellung dieser hemokompatiblen Oberflächen sind in den Ansprüchen 20 - 31 angegeben. Bevorzugte Ausführungsformen finden sich in den abhängigen Ansprüchen, den Beispielen sowie den Figuren.

Gegenstand der vorliegenden Erfindung sind Medizinprodukte, deren Oberfläche zumindest teilweise mit einer hemokompatiblen Schicht versehen ist, wobei die hemokompatible Schicht mindestens eine Verbindung der Formel I umfaßt: 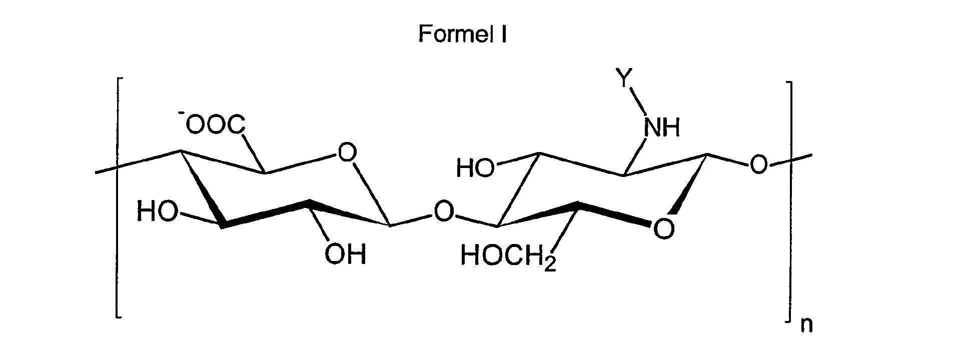 worin
n eine ganze Zahl zwischen 4 und 1050 bedeutet und
Y die Reste -CHO, -COCH₃, -COC₂H₅, -COC₃H₇, -COC₄H₉, -COC₅H₁₁, -COCH(CH₃)₂, -COCH₂CH(CH₃)₂, -COCH(CH₃)C₂H₅, -COC(CH₃)₃, -CH₂COO⁻, -C₂H₄COO⁻, -C₃H₆COO⁻, -C₄H₈COO⁻ darstellt.

Es können auch beliebige Salze der Verbindungen der Formel I verwendet werden. Die hemokompatible Schicht kann direkt auf die Oberfläche eines vorzugsweise nicht hemokompatiblen Medizinproduktes aufgebracht werden oder auf andere biostabile und/oder bioabbaubare Schichten aufgetragen werden. Ferner können sich auch auf der hemokompatiblen Schicht noch weitere biostabile und/oder bioabbaubare und/oder hemokompatible Schichten befinden. Zudem befindet sich auf, in und/oder unter der hemokompatiblen Schicht, bzw. den hemokompatiblen Schichten der Wirkstoff Paclitaxel. Der Wirkstoff (Paclitaxel) kann dabei eine eigene Wirkstoffschicht auf oder unter der hemokompatiblen Schicht bilden und/oder in mindestens einer der biostabilen, bioabbaubaren und/oder hemokompatiblen Schichten eingelagert werden. Bevorzugt werden die Verbindungen der allgemeinen Formel I verwendet, worin Y eine der folgenden Gruppen ist: -CHO, -COCH₃, -COC₂H₅ oder -COC₃H₇. Weiter bevorzugt sind die Gruppen -CHO, -COCH₃, -COC₂H₅ und insbesondere bevorzugt ist die Gruppe -COCH₃.

Die Verbindungen der allgemeinen Formel I enthalten nur noch einen geringen Anteil an freien Aminogruppen. Da mit dem Ninhydrintest keine freien Aminogruppen mehr nachgewiesen werden konnten, kann aufgrund der Empfindlichkeit dieses Tests geschlossen werden, dass weniger als 2%, bevorzugt weniger als 1% und insbesondere bevorzugt weniger als 0,5% aller -NH-Y-Gruppen als freie Aminogruppen vorliegen, d.h. bei diesem geringen Prozentsatz der Gruppen -NH-Y bedeutet Y Wasserstoff.

Da die Polysaccharide der allgemeinen Formel I Carboxylatgruppen und Aminogruppen enthalten, umfasst die allgemeine Formel auch Alkali- sowie Erdalkalimetallsalze der entsprechenden Polysaccharide. So können Alkalimetallsalze wie das Natriumsalz, das Kaliumsalz, das Lithiumsalz oder Erdalkalimetallsalze wie beispielsweise das Magnesiumsalz oder das Calciumsalz genannt werden. Ferner können mit Ammoniak, primären, sekundären, tertiären und quaternären Aminen, Pyridin und Pyridinderivaten Ammoniumsalze, bevorzugt Alkylammoniumsalze und Pyridiniumsalze gebildet werden. Zu den Basen, welche mit den Polysacchariden Salze bilden, zählen anorganische und organische Basen wie beispielsweise NaOH, KOH, LiOH, CaCO₃, Fe(OH)₃, NH₄OH, Tetraalkylammoniumhydroxide und ähnliche Verbindungen.

Die Polysaccharide gemäß Formel I weisen Molekulargewichte von 2kD bis 15kD auf, bevorzugt von 4kD bis 13kD, mehr bevorzugt von 6kD bis 12kD und insbesondere bevorzugt von 8kD bis 11 kD. Die Variable n ist eine ganze Zahl im Bereich von 4 bis 1050. Bevorzugt ist n eine ganze Zahl von 9 bis 400, mehr bevorzugt von 14 bis 260 und insbesondere bevorzugt eine ganze Zahl zwischen 19 und 210.

Die allgemeine Formel I gibt ein Disaccharid wieder, welches als Grundbaustein der verwendeten Polysaccharide anzusehen ist und durch n-fache Aneinanderreihung des Grundbausteins das Polysaccharid ergibt. Dieser aus zwei Zuckermolekülen aufgebaute Grundbaustein soll nicht dahingehend ausgelegt werden, daß unter die allgemeine Formel I nur Polysaccharide mit einer geraden Anzahl an Zuckermolekülen fallen. Natürlich umfaßt die Formel I auch Polysaccharide mit einer ungeraden Anzahl an Zuckerbausteinen. Als Endgruppen der Polysaccharide liegen Hydroxygruppen vor.

Insbesondere bevorzugt sind Medizinprodukte, welche unmittelbar auf der Oberfläche des Medizinproduktes eine hemokompatible Schicht aus den Verbindungen nach Formel I aufweisen und eine darüberliegende Schicht aus Paclitaxel. Die Paclitaxelschicht kann dabei teilweise in die hemokompatible Schicht diffundieren oder auch vollständig von der hemokompatiblen Schicht aufgenommen werden.

Ferner ist bevorzugt, wenn sich unter der hemokompatiblen Schicht zumindest eine biostabile Schicht befindet. Zudem kann die hemokompatible Schicht mit mindestens einer weiteren, darüberliegenden biostabilen und/oder bioabbaubaren Schicht vollständig oder/und unvollständig überzogen werden. Bevorzugt ist eine äußere bioabbaubare oder hemokompatible Schicht.

Eine weitere bevorzugte Ausführungsform weist eine Schicht aus Paclitaxel unter der hemokompatiblen Schicht auf oder zwischen der biostabilen und der hemokompatiblen Schicht, so dass das Paclitaxel durch die hemokompatible Schicht langsam freigesetzt wird. Das Paclitaxel kann kovalent und/oder adhäsiv in und/oder auf der hemokompatiblen Schicht und/oder der biostabilen und/oder der bioabbaubaren Schicht gebunden werden, wobei die adhäsive Bindung bevorzugt ist.

Als bioabbaubare Substanzen für die bioabbaubare(n) Schicht(en) können verwendet werden: Polyvalerolactone, Poly-ε-Decalactone, Polylactonsäure, Polyglycolsäure Polylactide, Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride wie Polymaleinsäure-anhydride, Polyhydroxymethacrylate, Fibrin, Polycyanoacrylate, Polycaprolactondimethylacrylate, Poly-b-Maleinsäure Polycaprolactonbutyl-acrylate, Multiblockpolymere wie z.B. aus Oligocaprolactondiole und Oligodioxanondiole, Polyetherestermultiblockpolymere wie z.B. PEG und Poly(butylenterephtalat. Polypivotolactone, Polyglycolsäuretrimethyl-carbonate Polycaprolacton-glycolide, Poly(g-ethylglutamat), Poly(DTH-Iminocarbonat), Poly(DTE-co-DT-carbonat), Poly(Bisphenol A-iminocarbonat), Polyorthoester, Polyglycol-säuretrimethyl-carbonate, Polytrimethylcarbonate Polyiminocarbonate, Poly(N-vinyl)-Pyrrolidon, Polyvinylalkohole, Polyesteramide, glycolierte Polyester, Polyphosphoester, Polyphosphazene, Poly[p-carboxyphenoxy)propan] Polyhydroxypentan-säure, Polyanhydride, Polyethylenoxid-propylenoxid, weiche Polyurethane, Polyurethane mit Aminosäurereste im Backbone, Polyetherester wie das Polyethylenoxid, Polyalkenoxalate, Polyorthoester sowie deren Copolymere, Lipide, Carrageenane, Fibrinogen, Stärke, Kollagen, protein-basierende Polymere, Polyaminosäuren, synthetische Polyaminosäuren, Zein, modifiziertes Zein, Polyhydroxyalkanoate, Pectinsäure, Actinsäure, modifiziertes und unmodifiziertes Fibrin und Casein, Carboxymethylsulfat, Albumin, desweiteren Hyaluronsäure, Chitosan und seine Derivate, Heparansulfate uns eine Derivate, Heparine, Chondroitinsulfat, Dextran, b-Cyclodextrine, und Copolymere mit PEG und Polypropylenglycol, Gummi arabicum, Guar, Gelatine, Collagen Collagen-N-Hydroxysuccinimid, Lipide, Phospholipide, Modifikationen und Copolymere und/oder Mischungen der vorgenannten Substanzen.

Als biostabile Substanzen für die biostabile(n) Schicht(en) können verwendet werden: Polyacrylsäure und Polyacrylate wie Polymethylmethacrylat, Polybutylmethacrylat, Polyacrylamid, Polyacrylonitrile, Polyamide, Polyetheramide, Polyethylenamin, Polyimide, Polycarbonate, Polycarbourethane, Polyvinylketone, Polyvinylhalogenide, Polyvinylidenhalogenide, Polyvinylether, Polyisobutylene, Polyvinylaromaten, Polyvinylester, Polyvinylpyrollidone, Polyoxymethylene, Polytetramethylenoxid, Polyethylen, Polypropylen, Polytetrafluorethylen, Polyurethane, Polyetherurethane Silicon-Polyetherurethane, Silicon-Polyurethane, Silicon-Polycarbonat-Urethane, Polyolefin-Elastomere, Polyisobutylene, EPDM-Gummis, Fluorosilicone, Carboxymethylchitosane, Polyaryletheretherketone, Polyetheretherketone, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseacetatbutyrate, Ethylvinylacetat-copolymere, Polysulfone, Epoxyharze, ABS-Harze, EPDM-Gummis, Silicone wie Polysiloxane, Polydimethylsiloxane, Polyvinylhalogene und Copolymere, Celluloseether, Cellulosetriacetate. Chitosane und Copolymere und/oder Mischungen dieser Substanzen.

Es können beliebige Medizinprodukte mit den hierin offenbarten hemokompatiblen Oberflächen versehen werden, insbesondere solche, die für den kurzzeitigen oder langzeitigen Kontakt mit Blut oder Blutprodukten geeignet sein sollen. Derartige Medizinprodukte sind beispielsweise Prothesen, Organe, Gefäße, Aorten, Herzklappen, Schläuchen, Organersatzteilen, Implantaten, Fasern, Hohlfasern, Stents, Kanülen, Spritzen, Membranen, Konserven, Blutbehältern, Titerplatten, Herzschrittmachern, Adsorbermedien, Chromatographiemedien, Chromatographiesäulen, Dialysatoren, Verbindungsstücke, Sensoren, Ventile, Zentrifugenkammern, Wäremaustauschern, Endoskope, Filter, Pumpenkammern. Insbesondere bezieht sich die vorliegende Erfindung auf Stents.

Die Polysaccharide der Formel I können aus Heparin und/oder Heparansulfaten hergestellt werden. Bei diesen Edukten handelt es sich um strukturell sehr ähnliche Verbindungen. Heparansulfate kommen ubiquitär auf Zelloberflächen von Säugetieren vor. Von Zelltyp zu Zelltyp unterscheiden sie sich stark in Molekulargewicht, Acetylierungsgrad und Sulfatierungsgrad. Leberheparansulfat weist beispielweise einen Acetylierungsgrad von ca. 50% auf, wohingegen das Heparansulfat aus der Glykokalix von Endothelzellen einen Acetylierungsgrad von bis zu 90% und größer aufweisen kann. Heparin weist nur einen sehr geringen Acetylierungsgrad von bis zu 5% auf. Der Sulfatierungsgrad liegt beim Leberheparansulfat und Heparin bei - 2 pro Disaccharideinheit, bei Endothelzellheparansulfat nahe bei 0 und bei Heparansulfaten aus anderen Zelltypen zwischen 0 und 2 pro Disaccharideinheit.

Die Verbindungen der allgemeinen Formel I zeichnen sich durch einen Gehalt an Sulfatgruppen pro Disaccharideinheit von weniger als 0,05 aus. Ferner beträgt der Gehalt an freien Aminogruppen in diesen Verbindungen weniger als 1 % bezogen auf sämtliche -NH-Y-Gruppen beträgt.

Die folgende Abbildung zeigt eine Tetrasaccharideinheit eines Heparins oder Heparansulfates mit statistischer Verteilung der Sulfatgruppen und einem Sulfatierungsgrad von 2 pro Disaccharideinheit wie er für Heparin typisch ist:

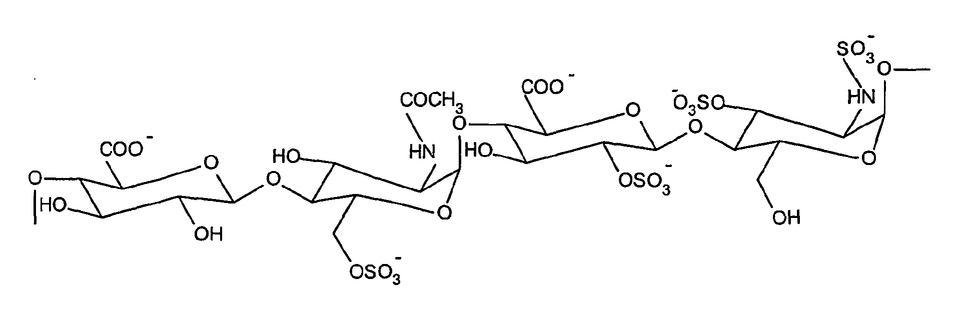

Allen Heparansulfaten ist mit Heparin der Ablauf der Biosynthese gemeinsam. Dabei wird als erstes das Coreprotein mit der Xylose-haltigen Bindungsregion aufgebaut. Sie besteht aus der Xylose und zwei damit verbundenen Galactoseresten. An den letzten der beiden Galactosereste wird dann abwechselnd je eine Glucuronsäure und ein Galactosamin gebunden, bis die entsprechende Kettenlänge erreicht ist. Abschließend erfolgt eine mehrstufige enzymatische Modifizierung dieses gemeinsamen Vorläufer-Polysaccharides aller Heparansulfate und des Heparins durch Sulfotransferasen und Epimerasen, die durch ihre unterschiedlich vollständigen Umsetzungen das breite Spektrum an verschiedenen Heparansulfaten bis hin zum Heparin generieren.

Heparin ist alternierend aus D-Glucosamin und D-Glucuronsäure bzw. L-Iduronsäure aufgebaut, wobei der Anteil an L-Iduronsäure bis zu 75% beträgt. D-Glucosamin und D-Glucuronsäure sind -1,4-glykosidisch bzw. L-Iduronsäure in α-1,4-glykosidischer Verknüpfung zum Disaccharid verknüpft, welches die Heparinuntereinheiten bildet. Diese Untereinheiten sind wiederum miteinander β-1,4-glykosidisch verknüpft und führen zum Heparin. Die Stellung der Sulfonylgruppen kann wechseln. Eine Tetrasaccharideinheit enthält durchschnittlich 4 bis 5 Schwefelsäurereste.
Heparansulfat, auch als Heparitinsulfat bezeichnet, enthält mit Ausnahme des Leberheparansulfates weniger N- und O-gebundene Sulfonylgruppen als Heparin, dafür aber mehr N-Acetylgruppen. Der Anteil an L-Iduronsäure ist im Vergleich zum Heparin ebenfalls geringer.

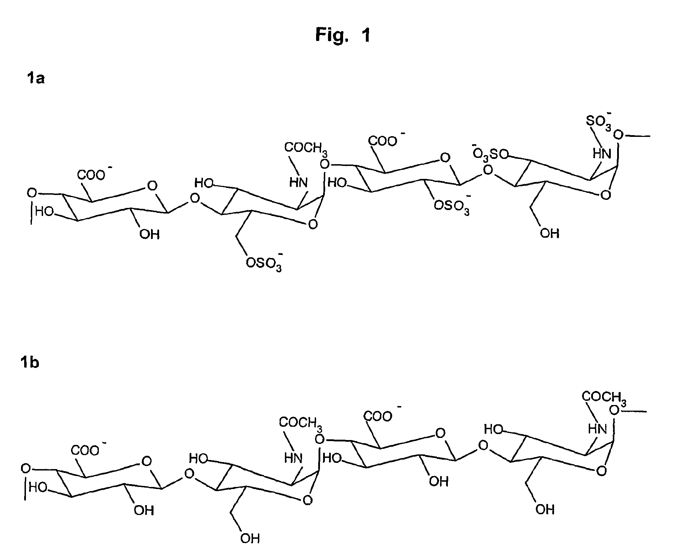

Wie aus Figur 1 deutlich wird, sind die Verbindungen der allgemeinen Formel (s. Figur 1b als Beispiel) dem natürlichen Heparansulfat der Endothelzellen strukturell ähnlich, vermeiden aber die eingangs geschilderten Nachteile bei der Verwendung von Endothelzellheparansulfaten.

Für die antithrombotische Wirksamkeit wird eine bestimmte Pentasaccharideinheit verantwortlich gemacht, die sich in handelsüblichen Heparinpräparaten in ca. jedem dritten Molekül wiederfindet. Durch spezielle Trennverfahren lassen sich Heparinpräparate unterschiedlicher antithrombotischer Aktivität herstellen. In hochaktiven, beispielsweise durch Antithrombin-III-Affinitätschromatographie erhaltenen Präparationen ("High-Affinity"-Heparin) findet sich diese aktive Sequenz in jedem Heparinmolekül, wogegen in "No-Affinity"-Präparationen keine charakteristischen Pentasaccharidsequenzen und damit keine aktive Gerinnungshemmung nachgewiesen werden können. Durch die Wechselwirkung mit diesem Pentasaccharid wird die Aktivität von Antithrombin III, einem Inhibitor des Gerinnungs-Schlüsselfaktors Thrombin, wesentlich potenziert (Bindungsaffinitätssteigerung bis um den Faktor 2X10³) [Stiekema J.C.J.; Clin Nephrology 26, Suppl. Nr 1, S3-S8, (1986)].

Die Aminogruppen des Heparins sind meistenteils N-sulfatiert oder N-acetyliert. Die wichtigsten O-Sulfatierungspositionen sind das C2 in der Iduronsäure, sowie das C6 und das C3 in Glucosamin. Für die Wirkung des Pentasaccharids auf die plasmatische Gerinnung wird allerdings hauptsächlich die Sulfatgruppe am C6 verantwortlich gemacht, in geringerem Maße auch die anderen funktionellen Gruppen.

Mit Heparin oder Heparansulfaten beschichtete Oberflächen medizinischer Implantate sind und bleiben durch die Beschichtung nur bedingt hemokompatibel. Das auf die künstliche Oberfläche aufgebrachte Heparin oder Heparansulfat verliert teilweise in drastischem Maße seine antithrombotische Wirkung, was mit einer aufgrund sterischer Hinderung eingeschränkten Wechselwirkung der besagten Pentasaccharideinheiten mit Antithrombin III in Zusammenhang gebracht wird.
In allen Fällen wird aufgrund der Immobilisierung dieser polyanionischen Substanzen eine starke Plasmaproteinadsorption auf der heparinisierten Oberfläche beobachtet, was einerseits die gerinnungsunterdrückende Wirkung des Heparins bzw. der Heparansulfate aufhebt und andererseits spezifische Gerinnungsvorgänge durch adhärierte und somit tertiärstrukturveränderte Plasmaproteine (z.B. Albumin, Fibrinogen, Thrombin) und darauf adhärierende Thrombozyten auslöst.

Somit besteht einerseits ein Zusammenhang zwischen der durch die Immobilisierung eingeschränkten Wechselwirkung der Pentasaccharideinheiten mit Antithrombin III, andererseits kommt es zu Ablagerungen von Plasmaproteinen auf der Heparin- bzw. Heparansulfatschicht auf dem medizinischen Implantat, was zum Verlust(en) der antithrombotischen Eigenschaften der Beschichtung führt und sich sogar ins Gegenteil umkehren kann, da die innerhalb weniger Sekunden eintretende Plasmaproteinadsorption zum Verlust der antikoagulierenden Oberfläche führt und die adhärierenden Plasmaproteine ihre Tertiärstruktur ändern, wodurch sich die Antithombogenität der Oberfläche sich ins Gegenteil kehrt und eine thrombogene Oberfläche entsteht. Es konnte überraschenderweise festgestellt werden, dass die Verbindungen der allgemeinen Formel trotz der strukturellen Unterschiede zum Heparin bzw. Heparansulfat die hemokompatiblen Eigenschaften von Heparin weiterhin aufweisen und zudem bei der Immobilisierung der Verbindungen keine nennenswerten Ablagerungen von Plasmaproteinen, die einen initialen Schritt in der Aktivierung der Gerinnungskaskade darstellen, beobachtet werden konnten. Die hemokompatiblen Eigenschaften der erfindungsgemäßen Verbindungen bleiben auch nach ihrer Immobilisierung auf künstlichen Oberflächen weiterhin erhalten.

Ferner wird vermutet, dass die Sulfatgruppen des Heparins bzw. der Heparansulfate für die Wechselwirkung mit Antithrombin III notwendig sind und dadurch dem Heparin bzw. Heparansulfat die antikoagulatorische Wirkung verleihen. Da durch eine weitgehend vollständige Desulfatierung die Sulfatgruppen bei den Verbindungen bis auf einen geringen Anteil von unter 0,2 Sulfatgruppen pro Disaccharideinheit entfernt werden, wirken die erfindungsgemäßen Verbindungen nicht selbst aktiv gerinnungsunterdrückend, d.h. antikoagulativ.

Die erfindungsgemäßen Verbindungen der allgemeinen Formel können aus Heparin oder Heparansulfaten hergestellt werden, indem zuerst das Polysaccharid im wesentlichen vollständig desulfatiert und danach im wesentlichen vollständig N-acyliert wird. Der Begriff "im wesentlichen vollständig desulfatiert" steht für einen Desulfatierungsgrad von größer 90%, bevorzugt größer 95% und besonders bevorzugt größer 98%. Der Desulfatierungsgrad kann gemäß dem sogenannten Ninhydrintest bestimmt werden, der freie Aminogruppen nachweist. Die Desulfatierung erfolgt in dem Maße, daß mit DMMB (Dimethylmethylenblau) keine Farbreaktion mehr erhalten wird. Dieser Farbtest ist zum Nachweis sulfatierter Polysaccharide geeignet, seine Nachweisgrenze ist in der Fachliteratur jedoch nicht bekannt. Die Desulfatierung kann beispielsweise durch Erhitzen des Pyridiniumsalzes in einem Lösungsmittelgemisch durchgeführt werden. Insbesondere hat sich eine Mischung von DMSO, 1,4-Dioxan und Methanol bewährt.

Heparansulfate sowie Heparin wurden durch Totalhydrolyse desulfatiert und anschließend reacyliert. Danach wurde die Zahl der Sulfatgruppen pro Disaccharideinheit (S/D) mittels C¹³-NMR bestimmt. Die folgende Tabelle 1 gibt diese Resultate am Beispiel von Heparin und desulfatiertem, reacetyliertem Heparin (Ac-Heparin) wieder.

**Tab. 1 : Verteilung der funktionellen Gruppen pro Disaccharideinheit am Beispiel von Heparin und Ac-Heparin bestimmt mittels ¹³C-NMR-Messungen**

| | **2-S** | **6-S** | **3-S** | **NS** | **N-Ac** | **NH₂** | **S/D** |
|---|---|---|---|---|---|---|---|
| Heparin | 0,63 | 0,88 | 0,05 | 0,90 | 0,08 | 0,02 | 2,47 |
| Ac-Heparin | 0,03 | 0 | 0 | 0 | 1,00 | - | 0,03 |

| | | | | | | | |
|---|---|---|---|---|---|---|---|
| 2-S, 3-S, 6-S: Sulfatgruppen am Position 2, 3 bzw. 6 NS: Sulfatgruppen an den Aminogruppen N-Ac: Acetylgruppen an den Aminogruppen NH₂: freie Aminogruppen S/D: Sulfatgruppen pro Disaccharideinheit | | | | | | | |

Reproduzierbar ergab sich ein Sulfatgehalt von ca. 0,03 Sulfatgruppen/Disaccharideinheit (S/D) beim Ac-Heparin im Vergleich mit ca. 2,5 Sulfatgruppen/Disaccharideinheit beim Heparin

Wie oben beschrieben hat der Unterschied in den Sulfatgehalten von Heparin bzw. Heparansulfaten einen wesentlichen Einfluss auf die Aktivität gegenüber Antithrombin III und der antikoagulativen Wirkung dieser Verbindungen. Sie weisen einen Gehalt an Sulfatgruppen pro Disaccharideinheit von weniger als 0,2, bevorzugt weniger als 0,07, weiter bevorzugt weniger als 0,05 und insbesondere bevorzugt weniger als 0,03 Sulfatgruppen pro Disaccharideinheit auf.

Durch die Entfernung der Sulfatgruppen des Heparins, denen die aktiven gerinnungsunterdrückenden Wirkungsmechanismen zugeschrieben werden, erhält man ein zur Oberflächenveredlung geeignetes hemokompatibles, gerinnungsinertes Oligo- bzw. Polysaccharid, das einerseits keine aktive Rolle im Gerinnungsprozess mehr einnimmt, und das andererseits vom Gerinnungssystem nicht als Fremdoberfläche erkannt wird. Diese Beschichtung imitiert erfolgreich den von der Natur vorgegebenen Höchststandard der Hemokompatibilität und Passivität gegenüber den gerinnungsaktiven Komponenten des Blutes. Die Beispiele 3 und 4 verdeutlichen, dass Oberflächen, welche mit den erfindungsgemäßen Verbindung beschichtet werden, insbesondere kovalent beschichtet werden, eine passivierende, athrombogene und hemokompatible Beschichtung ergeben. Dies wird eindeutig am Beispiel des Ac-Heparins belegt.

Im wesentlichen vollständig N-acyliert bezieht sich auf einen N-Acylierungsgrad von größer 94%, bevorzugt größer 97% und besonders bevorzugt größer 98%. Die Acylierung verläuft derart vollständig, daß mit dem Ninhydrinnachweis auf freie Aminogruppen keine Farbreaktion mehr erhalten wird. Als Acylierungsmittel werden bevorzugt Carbonsäurechloride, -bromide oder -anhydride eingesetzt. Essigsäureanhydrid, Propionsäureanhydrid, Buttersäureanhydrid, Essigsäurechlorid, Propionsäurechlorid oder Buttersäurechlorid eigenen sich beispielsweise zur Herstellung der erfindungsgemäßen Verbindungen. Insbesondere eignen sich Carbonsäureanhydride als Acylierungsmitttel.
Als Lösungsmittel insbesondere für die Carbonsäureanhydride wird deionisiertes Wasser verwendet, bevorzugt zusammen mit einem Cosolvens, welches in einer Menge von 10 bis 30 Volumenprozent beigesetzt wird. Als Cosolventien eignen sich Methanol, Ethanol, DMSO, DMF, Aceton, Dioxan, THF, Essigsäureethylester und andere polare Lösungsmittel. Bei der Verwendung von Carbonsäurehalogeniden werden bevorzugt polare wasserfreie Lösungsmittel wie DMSO oder DMF eingesetzt.

Als Lösungsmittel wird deionisiertes Wasser verwendet, bevorzugt zusammen mit einem Cosolvens, welches in einer Menge von 10 bis 30 Volumenprozent beigesetzt wird. Als Cosolventien eignen sich Methanol, Ethanol, DMSO, DMF, Aceton, Dioxan, THF, Essigsäureethylester und andere polare Lösungsmittel.

Die erfindungsgemäßen Verbindungen der allgemeinen Formel weisen an der Hälfte der Zuckermoleküle eine Carboxylatgruppe und an der anderen Hälfte eine N-Acylgruppe auf.

Die vorliegende Erfindung beschreibt die Verwendung der Verbindungen der allgemeinen Formel sowie Salze dieser Verbindungen für die Beschichtung, insbesondere eine hemokompatible Beschichtung von natürlichen und/oder künstlichen Oberflächen. Unter "hemokompatibel" wird die Eigenschaft der erfindungsgemäßen Verbindungen verstanden, nicht mit den Stoffen des Blutgerinnungssystems oder den Blutplättchen wechselzuwirken und daher nicht die Blutgerinnungskaskade auszulösen.

Zudem offenbart die Erfindung Polysaccharide für die hemokompatible Beschichtung von Oberflächen. Bevorzugt sind Polysaccharide innerhalb der oben genannten Molekulargewichtsgrenzen. Die verwendeten Polysaccharide zeichnen sich dadurch aus, daß sie den Zuckerbaustein N-Acylglucosamin in großer Menge enthalten. Dies bedeutet, daß 40 bis 60% der Zuckerbausteine N-Acylglucosamin sind und im wesentlichen die restlichen Zuckerbausteine jeweils einen Carboxylrest aufweisen. Die Polysaccharide bestehen somit gewöhnlich zu über 95%, bevorzugt zu über 98%, aus nur zwei Zuckerbausteinen, wobei ein Zuckerbaustein einen Carboxylrest und der andere einen N-Acylrest trägt.

Ein Zuckerbaustein der Polysaccharide ist N-Acylglucosamin bevorzugt N-Acetylglucosamin und bei dem anderen handelt es sich um die Uronsäuren, Glucuronsäure und Iduronsäure.
Bevorzugt sind Polysaccharide, welche im wesentlichen aus dem Zucker Glucosamin bestehen, wobei im wesentlichen die Hälfte der Zuckerbausteine eine N-Acylgruppe trägt, bevorzugt eine N-Acetylgruppe, und die andere Hälfte der Glucosaminbausteine eine über die Aminogruppe direkt oder über eine oder mehrere Methylenylgruppen gebundene Carboxylgruppe trägt. Bei diesen an die Aminogruppe gebundenen Carbonsäurereste handelt es sich bevorzugt um Carboxymethyl- oder Carboxyethylgruppen. Ferner sind Polysaccharide bevorzugt, welche im wesentlichen zur Hälfte aus N-Acylglucosamin, bevorzugt aus N-Acetylglucosamin und im wesentlichen zur anderen Hälfte aus den Uronsäuren Glucuronsäure und Iduronsäure bestehen. Besonders bevorzugt sind die Polysaccharide, welche eine im wesentlichen alternierende Abfolge von N-Acylglucosamin und einer der beiden Uronsäuren aufweisen.

Überraschenderweise hat sich gezeigt, daß sich für die erfindungsgemäßen Verwendungen insbesondere im wesentlichen desulfatiertes und im wesentlichen N-acyliertes Heparin besonders gut eignet. Insbesondere sind für die hemokompatible Beschichtung N-acetyliertes Heparin geeignet.

Der Begriff "im wesentlichen" soll verdeutlichen, daß statistische Abweichungen zu berücksichtigen sind. Eine im wesentlichen alternierende Abfolge der Zuckerbausteine besagt, daß in der Regel keine zwei gleichen Zuckerbausteine aneinander gebunden sind, schließt aber eine derartige Fehlverknüpfung nicht vollkommen aus. Entsprechend bedeutet "im wesentlichen zur Hälfte" annähernd 50%, läßt aber geringe Schwankungen zu, da gerade bei biosynthetisch hergestellten Makromolekülen nie der Idealfall erreicht wird und immer gewisse Abweichungen berücksichtigt werden müssen, da Enzyme nicht perfekt arbeiten und bei der Katalyse mit einer gewissen Fehlerrate zu rechnen ist. Im Falle des natürlichen Heparins liegt hingegen eine streng alternierende Abfolge von N-Acetylglucosamin- und den Uronsäureeinheiten vor.

Des weiteren werden Verfahren zur hemokompatiblen Beschichtung von Oberflächen offenbart, welche insbesondere für den direkten Blutkontakt bestimmt sind. Bei diesem Verfahren wird eine natürliche und/oder künstliche Oberfläche bereitgestellt und die oben beschriebenen Polysaccharide werden auf dieser Oberfläche immobilisiert.
Die Immobilisierung der Polysaccharide auf diesen Oberflächen kann mittels hydrophober Wechselwirkungen, van der Waals Kräften, elektrostatischer Wechselwirkungen, Wasserstoffbrücken, ionischer Wechselwirkungen, Quervernetzung der Polysaccharide und/oder durch kovalente Bindung an die Oberfläche bewirkt werden. Bevorzugt ist die kovalente Verknüpfung der Polysaccharide (side-on Bindung), mehr bevorzugt die kovalente Einzelpunktverknüpfung (side-on Bindung) und insbesondere bevorzugt die kovalente Endpunktverknüpfung (end-on Bindung).

Im folgenden werden die erfindungsgemäßen Beschichtungsverfahren beschrieben. Biologische und/oder künstliche Oberflächen von Medizinprodukten können nach folgendem Verfahren mit einer hemokompatiblen Beschichtung versehen werden:
a) Bereitstellen einer Oberfläche eines Medizinprodukts und
b) Aufbringen mindestens einer Verbindung der allgemeinen Formel I gemäß Anspruch 1 als hemokompatible Schicht auf diese Oberfläche und/oder
b') Aufbringen einer biostabilen und/oder bioabbaubaren Schicht auf die Oberfläche des Medizinprodukts oder der hemokompatiblen Schicht.

Unter "Aufbringen" soll die zumindest teilweise Beschichtung einer Oberfläche mit den entsprechenden Verbindungen verstanden werden, wobei die Verbindungen auf und/oder in die darunterliegende Oberfläche auf- und/oder eingebracht und/oder immobilisiert oder sonstwie verankert werden.

Unter "im wesentlichen die restlichen Zuckerbausteine" ist zu verstehen, dass 93% der restlichen Zuckerbausteine, bevorzugt 96% und insbesondere bevorzugt 98% der restlichen 60% - 40% der Zuckerbausteine einen Carboxylrest tragen.
Bevorzugt wird eine unbeschichtete und/oder nicht hemokompatible Oberfläche bereitgestellt. Unter "nicht hemokompatible" Oberflächen sollen solche Oberflächen verstanden werden, welche das Blutgerinnungssystem aktivieren können, also mehr oder minder thrombogen sind.

Eine alternative Ausführungsform umfaßt die Schritte:
a) Bereitstellen einer Oberfläche eines Medizinprodukts und
b) Aufbringen mindestens eines erfindungsgemäßen Polysaccharids nach Formel I,
b') Aufbringen einer biostabilen Schicht auf die Oberfläche des Medizinprodukts und
d') Aufbringen einer weiteren hemokompatiblen Schicht aus mindestens einem erfindungsgemäßen Polysaccharid nach Formel I.

Die letztgenannte Ausführungsform stellt selbst bei z.B. mechanischer Beschädigung der polymeren Schicht und damit auch der äußeren hemokompatiblen Schicht sicher, dass die Oberflächenbeschichtung ihre Eigenschaft, blutverträglich zu sein, nicht verliert.

Unter "biologische und künstliche" Oberfläche ist die Kombination eines künstlichen Medizinprodukts mit einem künstlichen Teil zu verstehen, z.B. ein Schweineherz mit einer künstlichen Herzklappe.

Die einzelnen Schichten werden bevorzugt durch Tauch- oder Sprühverfahren aufgetragen, wobei man zugleich mit der Auftragung einer Schicht auch Paclitaxel auf die Medizinproduktoberfläche aufbringen kann, welches dann in der jeweiligen Schicht kovalent und/oder adhäsiv gebunden enthalten ist. So kann zugleich mit dem Aufbringen einer hemokompatiblen Schicht auf dem Medizinprodukt auch der Wirkstoff Paclitaxel aufgebracht werden. Die Substanzen für die biostabilen oder bioabbaubaren Schichten wurden bereits oben aufgeführt.

Auf diese erste biostabile und/oder bioabbaubare oder hemokompatible Schicht kann dann in einem weiteren nicht zwingenden Schritt c) eine Wirkstoffschicht aus Paclitaxel aufgetragen werden. In einer bevorzugten Ausführungsform wird Paclitaxel kovalent an die darunterliegende Schicht gebunden. Auch Paclitaxel wird bevorzugt im Tauch- oder Sprühverfahren auf und/oder in die hemokompatible Schicht oder die biostabile Schicht auf- und/oder eingebracht.

Nach dem Schritt b) oder dem Schritt c) kann ein weiterer Schritt d) folgen, welcher das Aufbringen mindestens einer bioabbaubaren Schicht und/oder mindestens einer biostabilen Schicht auf die hemokompatible Schicht bzw. die Schicht aus Paclitaxel umfaßt.

Entsprechend der alternativen Ausführungsform kann nach Schritt b') oder Schritt c) ein Schritt d') folgen, welcher das Aufbringen mindestens einer Verbindung der allgemeinen Formel I als hemokompatible Schicht auf die biostabile und/oder bioabbaubare Schicht bzw. die Schicht auf Paclitaxel, umfaßt. Bevorzugt folgt nach Schritt b') der Schritt d').

Nach Schritt d) bzw. d') kann das Aufbringen von Paclitaxel in und/oder auf die mindestens eine bioabbaubare und/oder biostabile Schicht oder die hemokompatible Schicht erfolgen. Die einzelnen Schichten sowie Paclitaxel werden bevorzugt im Tauch- oder Sprühverfahren auf und/oder in die darunterliegende Schicht auf- und/oder eingebracht.

Gemäß einer bevorzugten Ausführungsform wird die biostabile Schicht auf die Oberfläche des Medizinprodukts aufgetragen und vollständig oder unvollständig mit einer hemokompatiblen Schicht überzogen, welche (bevorzugt kovalent) an die biostabile Schicht gebunden wird.

Bevorzugt besteht die hemokompatible Schicht aus Heparin nativen Ursprungs regioselektiv hergestellter Derivate unterschiedlicher Sulfatierungsgrade und Acylierungsgrade im Molekulargewichtsbereich des für die antithrombotische Wirkung verantwortlichen Pentasaccharides bis zum Standardmolekulargewicht des käuflichen Heparins von ca. 13kD, Heparansulfate und seine Derivate, Oligo- und Polysaccharide der Erythrocytenglycocalix, desulfatiertes und N-reacetyliertes Heparin, N-carboxymethyliertes und/oder partiell N-acetyliertes Chitosan sowie aus Mischungen dieser Substanzen.

Gegenstand der Erfindung sind auch die Medizinprodukte, welche nach einem der hierin genannten Verfahren hemokompatibel beschichtet worden sind. Bei den Medizinprodukten handelt es sich vorzugsweise um Stents.

Die herkömmlichen Stents, welche gemäß der erfindungsgemäßen Verfahren beschichtet werden können, bestehen aus Edelstahl, Nitinol oder anderen Metallen und Legierungen oder aus synthetischen Polymeren.

Die erfindungsgemäßen Stents sind mit einer nach der allgemeinen Formel bevorzugt kovalent gebundenen hemokompatiblen Schicht überzogen. Eine zweite Schicht bedeckt diese erste hemokompatible Schicht vollständig oder auch unvollständig. Diese zweite Schicht besteht vorzugsweise aus Paclitaxel. Die hemokompatible Beschichtung eines Stents sorgt einerseits für die notwendige Blutverträglichkeit und senkt damit das Thromboserisiko und ebenso für die Eindämmung der durch den Eingriff und durch die Anwesenheit einer körperfremden Oberfläche hervorgerufenen Entzündungsreaktionen und Paclitaxel, der bevorzugt über die Gesamtoberfläche des Stents gleichmäßig verteilt ist, bewirkt, daß der Bewuchs der Stentoberfläche mit Zellen, insbesondere glatten Muskel- und Endothelzellen, in einer kontrollierten Weise abläuft, so daß das Zusammenspiel von Thrombosereaktionen und Entzündungsreaktionen, Auschüttung von Wachstumsfaktoren, Zellproliferation und -migration während des Heilungsprozesses die Entstehung einer neuen "reparierten" Zellschicht bewirkt, die als Neointima bezeichnet wird.

Somit gewährleistet die Verwendung von Paclitaxel, kovalent oder/und adhäsiv an die darunterliegende Schicht gebunden oder/und kovalent oder/und adhäsiv in mindestens eine Schicht eingelagert, dass kontinuierlich und in geringen Dosen dieser Wirkstoff freigesetzt wird, so dass nicht die Besiedlung der Stentoberfläche mit Zellen unterbunden, jedoch eine Überbesiedlung und das Einwachsen der Zellen in das Gefäßlumen verhindert wird. Diese Kombination beider Effekte verleiht dem erfindungsgemäßen Stent die Fähigkeit, schnell in die Gefäßwand einzuwachsen und vermindert sowohl das Risiko einer Restenose als auch das Risiko einer Thrombose. Die Freisetzung von Paclitaxel erstreckt sich über einen Zeitraum von 1 bis 12 Monaten, bevorzugt über 1-3 Monate nach Implantation.

Paclitaxel ist bevorzugt in einer pharmazeutisch aktiven Konzentration von 0,001-10 mg pro cm² Stentoberfläche, bevorzugt 0,01 - 5 mg und insbesondere bevorzugt 0,1 - 1,0 mg pro cm² Stentoberfläche enthalten. Weitere Wirkstoffe können in ähnlicher Konzentration in derselben oder in der hemokompatiblen Schicht enthalten sein.

Die aufgetragenen Polymermengen liegen pro Schicht zwischen 0,01 mg bis 3 mg, bevorzugt 0,20 mg bis 1 mg und insbesondere bevorzugt zwischen 0,2 mg bis 0,5 mg.
Derartig beschichtete Stents setzen den Wirkstoff Paclitaxel kontrolliert und kontinuierlich frei und eigenen sich daher hervorragend zur Verhinderung und Verminderung von Restenose.

Diese Stents mit einer hemokompatiblen Beschichtung werden hergestellt, indem man Stents bereitstellt und bevorzugt kovalent eine der allgemeinen Formel entsprechende hemokompatible Schicht aufbringt, welche nach Wirkstofffreigabe und damit nach Abklingen des Wirkstoffeinflusses die Oberfläche des Implantates permanent maskiert.

Die bevorzugte Ausführungsform der erfindungsgemäßen Stents weist eine Beschichtung auf, welche aus mindestens zwei Schichten besteht. Dabei wird als zweite Schicht diejenige Schicht bezeichnet, welche auf die erste Schicht aufgebracht wird. Gemäß der Zweischichtausführung besteht die erste Schicht aus der hemokompatiblen Schicht, welche im wesentlichen vollständig von einer zweiten Schicht überzogen ist, die aus Paclitaxel besteht, das kovalent oder/und adhäsiv an die 1. Schicht gebunden ist.

Die Paclitaxelschicht wird langsam gelöst, so daß der Wirkstoff entsprechend der Geschwindigkeit des Lösungsvorgangs freigesetzt wird. Die erste hemokompatible Schicht gewährleistet die notwendige Blutverträglichkeit des Stents, in dem Maße wie der Wirkstoff entfernt wird. Durch die Wirkstofffreisetzung wird ein Anwachsen von Zellen nur für eine gewisse Zeit stark reduziert und ein gezieltes kontrolliertes Anwachsen dort ermöglicht, wo die äußere Schicht bereits weitgehend abgebaut worden ist. Schlussendlich bleibt die hemokompatible Schicht als athrombogene Oberfläche erhalten und maskiert die Fremdoberfläche derart, dass weiterhin keine lebensbedrohliche Reaktion eintreten kann.

Derartige Stents lassen sich durch ein Verfahren zur hemokompatiblen Beschichtung von Stents herstellen, dem folgendes Prinzip zugrundeliegt :
a. Bereitstellen eines Stents
b. Aufbringen einer bevorzugt kovalent gebundenen hemokompatiblen Schicht
c. im wesentlichen vollständiges Überziehen der hemokompatiblen Schicht im Tauch- oder Sprühverfahren mit dem antiproliferativen Wirkstoff Paclitaxel.

Die erfindungsgemäßen Stents lösen sowohl das Problem der akuten Thrombose als auch das Problem der Neointimahyperplasie nach einer Stentimplantation. Zudem eignen sich die erfindungsgemäßen Stents aufgrund ihrer Beschichtung besonders gut zur kontinuierlichen Freisetzung eines oder mehrerer antiproliferativer, immunsuppressiver Wirkstoffe. Aufgrund dieser Fähigkeit der gezielten kontinuierlichen Wirkstofffreisetzung in erforderlicher Menge verhindern die erfindungsgemäß beschichteten Stents die Gefahr der Restenose fast vollständig.

Die natürlichen und/oder künstlichen Oberflächen, welche nach dem oben beschriebenen Verfahren mit einer hemokompatiblen Schicht der besagten Polysaccharide überzogen worden sind, eignen sich insbesondere als Implantate bzw. Organersatzteile, welche in direktem Kontakt mit dem Blutkreislauf und Blut stehen, vorzugsweise in Form von Stents in Kombination mit einem antiproliferativen Wirkstoff, vorzugsweise Paclitaxel, zur Verhinderung der Restenose.

Die erfindungsgemäß beschichteten Medizinprodukte eignen sich insbesondere, jedoch nicht nur, für den direkten und dauerhaften Blutkontakt, sondern zeichnen sich überraschenderweise auch durch die Eigenschaft aus, die Anhaftung von Proteinen auf derartig beschichteten Oberflächen zu verringern oder gar zu verhindern. Die Ablagerung von Plasmaproteinen auf Fremdoberflächen in Kontakt mit Blut ist ein essentieller und initialer Schritt für das weitere Geschehen in Bezug auf die Erkennung und die einsetzenden Maßnahmen von Seiten des Blutsystems.

Dies ist beispielsweise wichtig bei der in vitro Diagnostik aus Körperflüssigkeiten. Somit verhindert oder zumindest verringert das Aufbringen der erfindungsgemäßen Beschichtung beispielsweise auf Mikrotiterplatten oder anderen Trägermedien, welche für diagnostische Nachweisverfahren eingesetzt werden, die unspezifische Ablagerung von Proteinen, welche die in der Regel empfindlichen Nachweisreaktionen stören und zu einer Verfälschung des Analyseresultates führen können.

Durch die Verwendung der erfindungsgemäßen Beschichtung auf Adsorbermedien oder Chromatographiemedien wird ebenfalls die unspezifische Ablagerung von Proteinen verhindert oder verringert, wodurch bessere Trennungen erreicht werden und Produkte von größerer Reinheit gewonnen werden können.

### Figurenbeschreibung

- Figur 1: zeigt eine Tetrasaccharideinheit eines Heparins oder Heparansulfates mit statistischer Verteilung der Sulfatgruppen und einem Sulfatierungsgrad von 2 pro Disaccharideinheit wie er für Heparin typisch ist (Figur 1 a). Zum Vergleich der strukturellen Ähnlichkeiten zeigt Figur 1b ein Beispiel einer Verbindung gemäß der allgemeinen Formel in der Beschreibung.

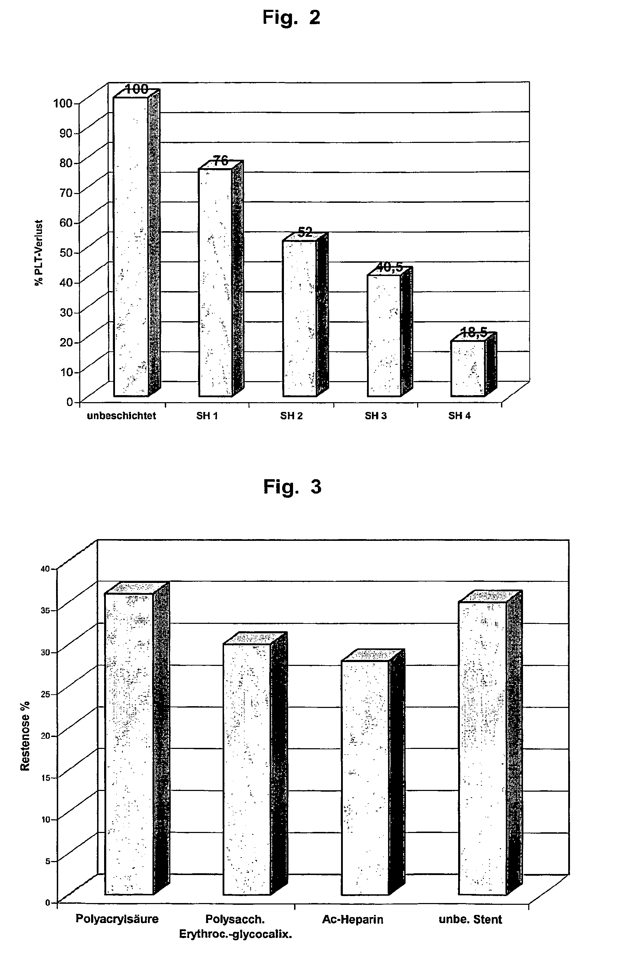

- Figur 2: zeigt den Einfluss von in einen PVC-Schlauch expandierten, oberflächenmodifizierten Edelstahl-Koronarstents auf den Thrombozytenverlust (PLT-Verlust). Als Referenz wurde ein unbeschichteter Edelstahl-Koronarstent vermessen (unbeschichtet). Als Nullwert wurde das Maß des Thrombozytenverlusts beim PCV-Schlauch ohne Edelstahl-Koronarstent angesetzt.
Dabei ist SH1 ist ein kovalent mit Heparin beschichteter Stent, SH2 ist ein mit Chondroitinsulfat beschichteter Stent; SH3 ist von der Erythrocytenglycocalix gewonnenen Polysacchariden beschichteter Stent und SH4 ist ein mit Ac-Heparin kovalent beschichteter Edelstahl-Koronarstent.
- Figur 3: zeigt eine bildliche Darstellung der Restenose-Rate von mit vollständig desulfatiertem und N-reacetyliertem Heparin (Ac-Heparin) kovalent beschichteten Stents und mit Oligo- und Polysacchariden der Erythrocytenglycocalix beschichteten Stents im Vergleich zum unbeschichteten Stent und mit Polyacrylsäure (PAS) beschichteten Stents nach 4 Wochen Implantationszeit im Schwein.

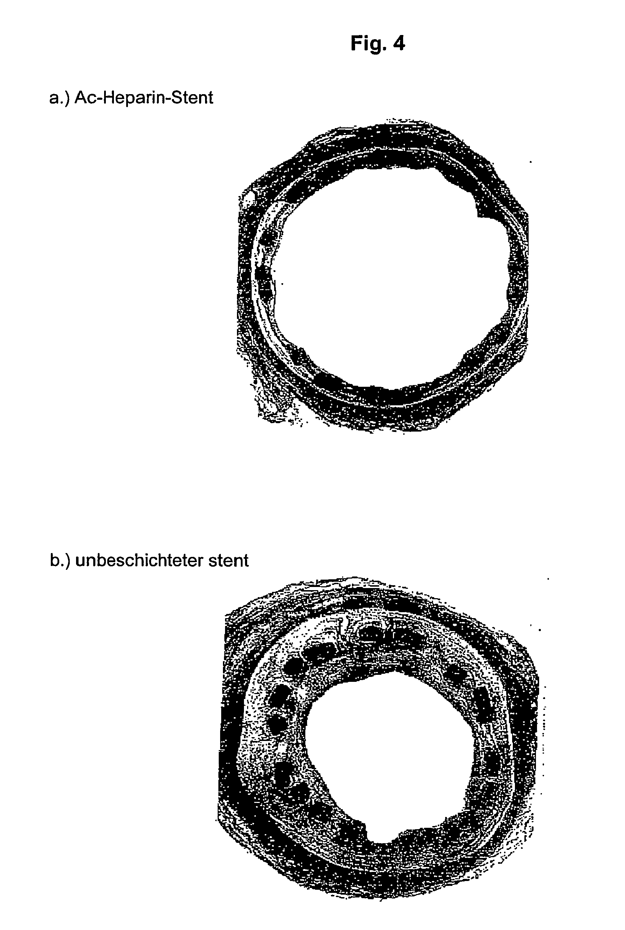

- Figur 4: Quantitative Koronarangiographie:
Aufnahmen der Querschnitte durch das den Stent enthaltende Gefäß-Segment eines mit Ac-Heparin beschichteten Stents (a.) und zum Vergleich die eines unbeschichteten (unbe.) Stents (b.). Nach vier Wochen im Tierversuch (Schwein) ist ein deutlicher Unterschied in den Dicken der gebildeten Neointimae zu erkennen.

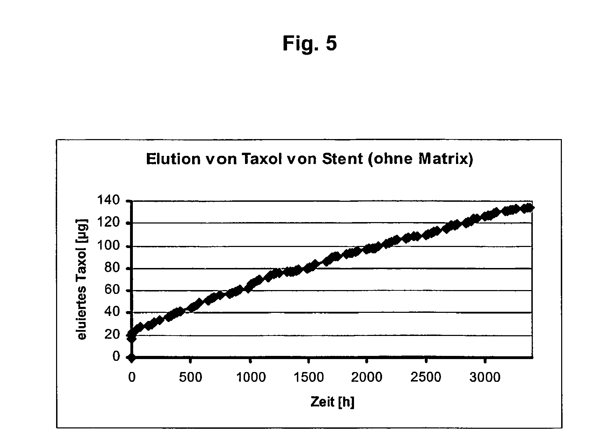

Figur 5 Elutionsdiagramm von Paclitaxel vom Stent (ohne Trägermaterial).

### Beispiele

### Beispiel 1

### Herstellung von desulfatiertem reacetyliertem Heparin:

100 ml Amberlite IR-122 Kationenaustauscherharz wurden in eine Säule mit 2 cm Durchmesser gefüllt, mit 400 ml 3M HCl in die H⁺-Form überführt und mit destilliertem Wasser gespült, bis das Eluat chloridfrei und pH neutral war. 1 g Natrium-Heparin wurde in 10 ml Wasser gelöst, auf die Kationenaustauschersäule gegeben und mit 400 ml Wasser eluiert. Das Eluat wurde in eine Vorlage mit 0,7 g Pyridin getropft und anschließend mit Pyridin auf pH 6 titriert und gefriergetrocknet.

0,9 g Heparin-Pyridinium-Salz wurden in einem Rundkolben mit Rückflußkühler mit 90 ml einer 6/3/1 Mischung aus DMSO/1,4-Dioxan/Methanol (V/V/V) versetzt und 24 Stunden auf 90°C erhitzt. Dann wurden 823 mg Pyridiniumchlorid zugegeben und weitere 70 Stunden auf 90°C erhitzt. Anschließend wurde mit 100 ml Wasser verdünnt und mit verdünnter Natronlauge auf pH 9 titriert. Das desulfatierte Heparin wurde gegen Wasser dialysiert und gefriergetrocknet.

100 mg des desulfatierten Heparins wurden in 10 ml Wasser gelöst, auf 0°C gekühlt und unter Rühren mit 1,5 ml Methanol versetzt. Zu der Lösung wurden 4 ml Dowex 1x4 Anionenaustauscherharz in der OH⁻-Form und anschließend 150 µl Essigsäureanhydrid gegeben und 2 Stunden bei 4°C gerührt. Danach wird das Harz abfiltriert und die Lösung gegen Wasser dialysiert und gefriergetrocknet.

### Beispiel 2

### Herstellung von desulfatiertem N-propionyliertem Heparin:

100 ml Amberlite IR-122 Kationenaustauscherharz wurden in eine Säule mit 2 cm Durchmesser gefüllt, mit 400 ml 3M HCl in die H⁺-Form überführt und mit destilliertem Wasser gespült, bis das Eluat chloridfrei und pH neutral war. 1 g Natrium-Heparin wurde in 10 ml Wasser gelöst, auf die Kationenaustauschersäule gegeben und mit 400 ml Wasser eluiert. Das Eluat wurde in eine Vorlage mit 0,7 g Pyridin getropft und anschließend mit Pyridin auf pH 6 titriert und gefriergetrocknet.

0,9 g Heparin-Pyridinium-Salz wurden in einem Rundkolben mit Rückflußkühler mit 90 ml einer 6/3/1 Mischung aus DMSO/1,4-Dioxan/Methanol (V/V/V) versetzt und 24 Stunden auf 90°C erhitzt. Dann wurden 823 mg Pyridiniumchlorid zugegeben und weitere 70 Stunden auf 90°C erhitzt. Anschließend wurde mit 100 ml Wasser verdünnt und mit verdünnter Natronlauge auf pH 9 titriert. Das desulfatierte Heparin wurde gegen Wasser dialysiert und gefriergetrocknet.

100 mg des desulfatierten Heparins wurden in 10 ml Wasser gelöst, auf 0°C gekühlt und unter Rühren mit 1,5 ml Methanol versetzt. Zu der Lösung wurden 4 ml Dowex 1x4 Anionenaustauscherharz in der OH -Form und anschließend 192 µl Propionsäureanhydrid gegeben und 2 Stunden bei 4°C gerührt. Danach wird das Harz abfiltriert und die Lösung gegen Wasser dialysiert und gefriergetrocknet.

### Beispiel 3

### Hemokompatibilitätbestimmungen von Verbindungen gemäß der allgemeinen Formel nach ISO 10933-4 (in vitro Untersuchungen)

Zur Untersuchung der Hemokompatibilität der Verbindungen gemäß der Formel I wurden Cellulosemembranen, Silikonschläuche und Edelstahlstents mit einer Verbindung gemäß Formel I kovalent beschichtet und gegen Heparin sowie gegen die entsprechenden, in den einzelnen Versuchen verwendeten unbeschichteten Material-Oberflächen getestet.

### 3.1. Cellulosemembranen (Cuprophan) beschichtet mit desulfatiertem, reacetyliertem Heparin (Ac-Heparin)

Zur Untersuchung der gerinnungsphysiologischen Wechselwirkungen zwischen citriertem Vollblut und den Ac-Heparin- bzw. Heparin-beschichteten Cuprophanmembranen wird das offene Perfusionssystem der von Sakariassen modifizierten Baumgartner-Kammer eingesetzt [Sakariassen K.S. et al.; J. Lab. Clin. Med. 102 : 522-535 (1983)]. Die sich aus vier Bauteilen zzgl. Dichtringen und Verschraubungen zusammensetzende Kammer ist aus Polymethylmethacrylat gefertigt und gestattet die parallele Untersuchung von zwei modifizierten Membranen, so dass in jeden Lauf bereits eine statistische Absicherung eingeht. Die Bauweise dieser Kammer erlaubt quasi-laminare Perfusionsbedingungen.

Nach 5-minütiger Perfusion bei 37°C werden die Membranen entnommen und nach Fixierung der adhärierten Blutplättchen die Thrombozytenbelegung bestimmt. Die jeweiligen Messwerte werden in Bezug zur hochthrombogenen Subendothelialen Matrix als Negativstandard mit einer 100%igen Plättchenbelegung gesetzt. Die Anlagerung der Thrombozyten erfolgt sekundär an die sich zuvor auf dem Fremdmaterial bildende Plasmaproteinschicht. Das Plasmaprotein Fibrinogen wirkt als Cofaktor der Plättchenaggregation. Die derart induzierte Aktivierung der Blutplättchen zieht die Bindung einer ganzen Reihe von gerinnungsassoziierten Plasmaproteinen, wie z.B. Vitronectin, Fibronectin und von Willebrand-Faktor auf der Thrombozytenoberfläche nach sich. Durch deren Einwirkung kommt es schließlich zur irreversiblen Aggregation der Thrombozyten. Die Thrombozytenbelegung stellt aufgrund der beschriebenen Wechselwirkungen ein anerkanntes Maß für die Thrombogenität von Oberflächen im Fall des Fremdoberflächenkontaktes von Blut dar. Aus dieser Tatsache ergibt sich die Schlussfolgerung: je geringer die Thrombozytenbelegung auf der perfundierten Oberfläche ist, als umso höher ist die Hemokompatibilität der untersuchten Oberfläche zu bewerten.

Die Ergebnisse der untersuchten Heparin-beschichteten und Ac-Heparin-beschichteten Membranen zeigen deutlich die Verbesserung der Hemokompatibilität der Fremdoberfläche durch die Beschichtung mit Ac-Heparin. Heparin-beschichtete Membranen zeigen eine 45 - 65%ige Thrombozytenbelegung, während Ac-Heparin-beschichtete Oberflächen Werte von 0 - 5% aufweisen (Bezug zur Subendothelialen Matrix mit 100%iger ThrombozytenBelegung).

Die Adhäsion der Thrombozyten auf der Ac-heparinisierten Oberfläche ist aufgrund der fehlenden, für die Aktivierung der Blutplättchen notwendigen, Plasmaproteine extrem erschwert. Im Gegensatz dazu bietet die Heparin- beschichtete Oberfläche mit der sofort einsetzenden Plasmaproteinadsorption optimale Voraussetzungen zur Aktivierung, Ablagerung und Aggregation der Thrombozyten; und letztendlich reagiert das Blut durch Aktivierung der Gerinnungskaskade mit den entsprechenden Abwehrmechanismen auf die eingesetzte Fremdoberfläche. Ac-Heparin erfüllt weitaus besser als Heparin die Ansprüche an die Hemokompatibilität der Fremdoberfläche.

Das Zusammenspiel von Plasmaproteinadsorption und Thrombozytenbelegung als direktes Maß für die Thrombogenität einer Oberfläche, in Abhängigkeit von der dem Blut dargebotenen Beschichtung, wird durch diesen in-vitro-Test besonders gut verdeutlicht. Demnach ist die Verwendung von kovalent gebundenem Heparin als antithrombotisch wirksame Oberfläche nur sehr eingeschränkt bis nicht möglich. Die Wechselwirkungen von immobilisiertem Heparin mit Blut kehren sich hier ins unerwünschte Gegenteil um - die heparinbeschichtete Oberfläche wird thrombogen.
Offensichtlich ist die herausragende Bedeutung von Heparin als Antithrombotikum nicht auf kovalent immobilisiertes Heparin übertragbar. In der systemischen Verabreichung kann es in gelöster Form seine Wirkung voll entfalten. Wird Heparin jedoch kovalent immobilisiert, ist seine antithrombotische Wirkung, wenn überhaupt, nur von kurzer Dauer. Anders das Ac-Heparin ("No-affinity"-Heparin), welches durch die Desulfatierung und N-Reacetylierung die aktiven antithrombotischen Eigenschaften des Ausgangsmoleküls zwar komplett verliert, dafür aber im Gegenzug ausgeprägte athrombogene Eigenschaften erwirbt, die nachweislich in der Passivität gegenüber Antithrombin III und der fehlenden Affinität gegenüber koagulationsinitiierenden Vorgängen begründet sind und nach kovalenter Bindung erhalten bleiben.

Dadurch eignet sich Ac-Heparin und damit die Verbindungen der allgemeinen Formel insgesamt bestens zur Tarnung von fremden Oberflächen im Kontakt mit dem Gerinnungssystem.

### 3.2. Immobilisierung auf Silikon:

Durch einen 1 m langen Silikonschlauch mit 3 mm Innendurchmesser wurde 30 Minuten lang bei 40°C 100 ml eines Gemisches aus Ethanol/Wasser 1/1 (V/V) im Kreis gepumpt. Dann wurden 2 ml 3-(Triethoxysilyl)-propylamin zugegeben und weitere 15 Stunden bei 40°C im Kreis gepumpt. Danach wurde noch jeweils 2 Stunden mit 100 ml Ethanol/Wasser und 100 ml Wasser gespült.

3 mg des deacetylierten und reacetylierten Heparins (Ac-Heparin) wurden bei 4°C in 30 ml 0,1 M MES-Puffer pH 4,75 gelöst und mit 30 mg CME-CDI (*N*-Cyclohexyl-*N*'-(2-morpholinoethyl)carbodiimidmethyl-p-toluolsulfonat) versetzt. Diese Lösung wurde für 15 Stunden bei 4°C im Kreis durch den Schlauch gepumpt. Anschließend wurde mit Wasser, 4M NaCl-Lösung und Wasser für je 2 Stunden gespült.

### 3.3 Bestimmung der Thrombozytenzahl (EN30993-4)

Auf einen 1 m langen Silikonschlauch mit 3 mm Innendurchmesser wurden zwei 2 cm lange enganliegende Glasröhrchen geschoben. Dann wurde der Schlauch mit einem Schrumpfschlauch zu einem Kreis geschlossen und luftfrei über Spritzen mit 0,154M NaCl Lösung gefüllt. Dabei wurde mit einer Spritze die Lösung eingefüllt und mit der anderen Spritze die Luft herausgezogen. Mit den beiden Spritzen wurde die Lösung luftblasenfrei gegen citriertes Vollblut eines gesunden Probanden ausgetauscht. Danach wurden die Einstichlöcher der Injektionsnadeln durch Überschieben der Glasröhrchen verschlossen und der Schlauch in eine Dialysepumpe gespannt. Das Blut wurde 10 Minuten mit einer Flußrate von 150 ml/min umgepumpt. Der Thrombozytengehalt des Blutes wurde vor und nach der Perfusion mit einem Coulter Counter bestimmt. Für unbeschichtete Silikonschläuche lag der Thrombozytenverlust bei 10%. Dagegen lag er bei Silikonschläuchen, die nach Beispiel 5.2 beschichtet wurden, im Durchschnitt bei 0% (Anzahl der Versuche: n=3).

Auch in diesem dynamischen Testsystem zeigt sich, das die Aktivierung von Thrombozyten an einer Oberfläche beschichtet mit Ac-Heparin reduziert ist. Gleichzeitig lässt sich festhalten, dass die Immobilisierung von Heparin aus den einen negativen Effekt auf die Hemokompatibilität der eingesetzten Oberfläche ausübt. Dagegen zeigt Ac-Heparin, seiner passiven Natur entsprechend, keine Effekte im Kontakt mit den Thrombozyten.

### 3.4. Vollblutuntersuchungen an 316 LVM-Edelstahl-Koronarstents

Im Rahmen der Biokompatibilitätsuntersuchungen wurden 31 mm lange 316 LVM-Edelstahl-Stents mit Ac-Heparin kovalent beschichtet. Bei einer Gesamtoberfläche von 2cm² und einer Belegungsdichte von ca. 20 pm/cm² Stentoberfläche beträgt die Beladung eines solchen Stents ca. 0,35 µg Ac-Heparin. Als Vergleich: Die bei der Thromboseprophylaxe übliche Tagesdosis von Heparin beträgt dagegen 20-30 mg und entspräche somit dem mindestens 60.000-fachen Wert.

Diese Untersuchungen wurden mit dem etablierten hämodynamischen Chandler-Loop-System durchgeführt [A. Henseler, B. Oedekoven, C. Andersson, K. Mottaghy; KARDIOTECHNIK 3 (1999)]. Beschichtete und unbeschichtete Stents wurden in PVC-Schläuchen (medical grade PVC) mit 600 mm Länge und 4 mm Innendurchmesser expandiert und getestet. Die Ergebnisse dieser Versuche bestätigen die bezüglich der Siliconschläuche diskutierten Untersuchungen. Der anfänglich auf den Stent zurückzuführende Blutplättchenverlust im Perfusat von 50% wird durch die Veredlung der Stentoberfläche mit Ac-Heparin um über 80% reduziert.

Der Einfluss von in den Schlauch expandierten, oberflächenmodifizierten Edelstahl-Koronarstents auf den Thrombozytenverlust wird in weiteren Chandler-Tests während der 45minütigen Vollblutperfusion evaluiert. Hierzu wird zuerst der stentfreie PVC-Schlauch untersucht, woraus sich der Nullwert ergibt. Der Leerschlauch zeigt einen mittleren Plättchenverlust von 27,4 % bezüglich des Spenderblutes bei einer Standardabweichung von lediglich 3,6%. Diesen Basiswert zugrundelegend werden unterschiedlich oberflächenmodifizierte Stents in den PVC-Schläuchen expandiert und unter analogen Bedingungen auf den von ihnen verursachten Plättchenverlust hin untersucht. Auch hier stellt sich wieder heraus, dass die durch den Stent bedeckte Oberfläche, die lediglich ca. 0,84% der gesamten Testoberfläche ausmacht, einen signifikanten und reproduzierbaren Effekt auf den Thrombozytengehalt verursacht. Bezogen auf den Leerschlauch (Basiswert) liefert die Untersuchung des polierten, chemisch nicht oberflächenvergüteten Stents einen zusätzlichen mittleren Thrombozytenverlust von 22,7%. Damit verursacht diese im Vergleich zum PVC-Leerschlauch weniger als 1 % messende Fremdoberfläche einen etwa vergleichbaren Thrombozytenverlust. Daraus leitet sich direkt ab, dass der als Stentmaterial verwendete medizinische Edelstahl 316 LVM, obwohl diese Testoberfläche nur 0,84% der gesamten Oberfläche ausmacht, verglichen mit einer medical grade PVC-Oberfläche, eine ca. 100-fach stärkere Plättchenschädigung induziert.

Die untersuchten Oberflächenvergütungen an den Edelstahl-Koronarstents zeigen sich in der Lage, das enorme Ausmaß der stentinduzierten Plättchenschädigung sehr deutlich zu reduzieren (siehe Fig. 2). Am effektivsten erwies sich mit 81,5% das Ac-Heparin (SH4).

Betrachtet man also die Auswirkungen der Ac-Heparin-beschichteten Stents auf den Thrombozytenverlust, ergeben sich gut übereinstimmende Werte. Die Korrelation der Thrombozytenverluste im Perfusat bzw. der Adhäsion der Thrombozyten an die dargebotenen Oberflächen zeigen die Verlässlichkeit der Ermittlungen.

### 3.4.1 Kovalente hemokompatible Beschichtung von Stents

Nicht expandierte Stents aus medizinischem Edelstahl LVM 316 wurden im Ultraschallbad 15 Minuten mit Aceton und Ethanol entfettet und bei 100°C im Trockenschrank getrocknet. Danach wurden sie für 5 Minuten in eine 2%ige Lösung von 3-Aminopropyltriethoxysilan in einem Gemisch Ethanol/Wasser (50/50 : (v/v)) getaucht und anschließend für 5 Minuten bei 100°C getrocknet. Anschließend wurden die Stents über Nacht mit demineralisiertem Wasser gewaschen.

3 mg desulfatiertes und reacetyliertes Heparin wurden bei 4°C in 30 ml 0,1M MES-Puffer (2-(N-Morpholino)ethansulfonsäure) pH 4,75 gelöst und mit 30 mg N-Cyclohexyl-N'-(2-morpholinoethyl)carbodiimid-methyl-p-toluolsulfonat versetzt. In dieser Lösung wurden 10 Stents für 15 Stunden bei 4°C gerührt. Anschließend wurde mit Wasser, 4M NaCl-Lösung und Wasser für je 2 Stunden gespült.

### 3.4.2 Bestimmung des Glucosamingehaltes der beschichteten Stents mittels HPLC

Hydrolyse : Die beschichteten Stents werden in kleine Hydrolyserohre gegeben und mit 3ml 3M HCl genau eine Minute bei Raumtemperatur stehengelassen. Die Metallproben werden entfernt und die Röhrchen nach dem Verschließen 16h im Trockenschrank bei 100°C inkubiert. Danach lässt man abkühlen, es wird dreimal zur Trockne eingedampft und in 1 ml entgastem und filtriertem Wasser aufgenommen und gegen einen ebenfalls hydrolysierten Standard in der HPLC vermessen:

| Stent | Fläche Probe | desulfat+reacet. Heparin [g/Probe] | Fläche [cm²] | desulfat+ reacet. Heparin [g/cm²] | desulfat+reacet. Heparin [pmol/cm²] |
|---|---|---|---|---|---|
| 1 | 129,021 | 2,70647E-07 | 0,74 | 3,65739E-07 | 41,92 |
| 2 | 125,615 | 2,63502E-07 | 0,74 | 3,56084E-07 | 40,82 |
| 3 | 98,244 | 1,93072E-07 | 0,74 | 2,60908E-07 | 29,91 |
| 4 | 105,455 | 2,07243E-07 | 0,74 | 2,80058E-07 | 32,10 |
| 5 | 119,061 | 2,33982E-07 | 0,74 | 3,16192E-07 | 36,24 |
| 6 | 129,202 | 2,53911E-07 | 0,74 | 3,43124E-07 | 39,33 |
| 7 | 125,766 | 2,53957E-07 | 0,74 | 3,43185E-07 | 39,34 |

### Beispiel 4

### In vivo Untersuchungen von beschichteten Koronarstents (Fig. 5)

### 4.1. In vivo Untersuchungen von Koronarstents beschichtet mit Ac-Heparin

Aufgrund der Daten zur Hemokompatibilität, die Ac-Heparin in den in-vitro-Versuchen geliefert hat, wurde die Eignung der Ac-Heparin-Oberfläche als athrombogene Beschichtung von Metallstents in vivo (im Tierversuch) untersucht.
Das Ziel der Versuche bestand primär darin, den Einfluß der Ac-Heparin-Beschichtung auf die stentinduzierte Gefäßreaktion zu bewerten. Neben dem Registrieren möglicher thrombotischer Ereignisse wurden die für restenotische Prozesse relevanten Parameter wie Neointimafläche, Gefäßlumen und Stenosegrad erfasst.
Für die Untersuchungen wurden 6 - 9 Monate alte Hausschweine, ein für die Bewertung von Stents seit langem etabliertes und anerkanntes Tiermodell, verwendet.

Erwartungsgemäß wurden in diesen Versuchen weder akute, subakute noch spätakute thrombotische Ereignisse registriert, was als Bestätigung der athrombogenen Eigenschaften des Ac-Heparins gewertet werden darf.

Nach vier Wochen wurden die Tiere euthanasiert, die gestenteten Koronararterien-Segmente entnommen und histomorphometrisch analysiert.
Hinweise auf eine mögliche akute oder subchronische Toxizität, Sensibilisierungsreaktionen oder anderweitige Irritationen als Folge der Implantation von Ac-Heparin-beschichteten Stents sind während der gesamten Versuchsphase, insbesondere bei der histologischen Auswertung, nicht festgestellt worden. Während der Stentimplantation sowie im Follow-up wurden koronar-angiographische Datensätze ermittelt, die eine Auswertung im Hinblick auf die Gefäßreaktion auf die Stentimplantation erlauben.
Der Unterschied zwischen dem unbeschichteten Kontrollstent und dem mit Ac-Heparin beschichteten Stent ist eindeutig. Die Ausbildung einer ausgeprägten Neointimaschicht ist bei dem unbeschichteten Kontrollstent sehr gut erkennbar. Schon nach vier Wochen zeigt sich die proliferationsfördernde Wirkung der unbeschichteten Stentoberfläche auf das umliegende Gewebe in einem solchen Ausmaß, dass letztendlich die Gefahr des Gefäßverschlusses im Stentbereich gegeben ist.

Im Fall des Ac-Heparin-beschichteten Stents lässt sich dagegen eine deutlich dünnere Neointimaschicht erkennen, die für ein reguliertes Einwachsen des Stents unter Erhalt eines weiten, freien Gefäßlumens spricht.

Die detaillierten histomorphometrischen und koronarangiographischen Daten untermauern diese Aussage, indem übereinstimmend festgestellt werden kann, dass durch die Ac-Heparin-Beschichtung (SH4) die Neointimahyperplasie ("Restenose") um ca. 17-20% im Vergleich zum unbeschichteten Kontrollstent zurückgedrängt wurde.
Dieses Ergebnis ist unerwartet und bemerkenswert zugleich. Sicher wird von einer athrombogenen Oberfläche nicht gefordert, zusätzlich zur Bereitstellung hemokompatibler Eigenschaften auch die zu einer Neointimahyperplasie führenden Prozesse zu beeinflussen, d.h. Restenosen zu verhindern.
Zum einen wird durch die dichte, dauerhafte Belegung der Stentoberfläche mit Ac-Heparin ein direkter Zellkontakt zur Metalloberfläche verhindert. Da in der Fachliteratur die Emission bestimmter Metallionen in das implantatnahe Gewebe als eine wahrscheinliche Ursache der Restenose diskutiert wird, könnte in einer von der Beschichtung bewirkten Unterbindung des direkten Metallkontaktes eine antirestenotische Wirksamkeit begründet sein.

Zum anderen ist ein derartiger positiver Nebeneffekt durchaus plausibel, weil auf einer passiven, athrombogenen Stentoberfläche mit dem Ausbleiben einer Thrombozytenaggregation auch die proliferativen Wirkungen der dabei freigesetzten Wachstumsfaktoren ausbleiben. Es fällt somit ein entscheidender, von der Lumenseite ausgehender Stimulus der neointimalen Proliferation aus.

### Beispiel 5

### Beschichtung der Stents mit Taxol nach dem Sprühverfahren

Die nach Beispiel 1 und Beispiel 2 vorbereiteten nicht expandierten Stents werden gewogen und horizontal auf eine dünne Metallstange ( d= 0,2mm) gehängt, die auf die Rotationsachse der Rotations- und Vorschubanlage gesteckt ist und mit 28 U/min rotiert. Die Stents werden so aufgebracht, das die Innenseite des Stents die Stange nicht berührt. Bei einer Vorschubamplitude von 2,2,cm und einer Vorschubgeschwindigkeit von 4cm/s und einem Abstand von 6 cm zwischen Stent und Düse wird der Stent mit der jeweiligen Sprühlösung besprüht. Nach dem Trocknen ( ca 15 min ) bei Raumtemperatur und folgend im Abzug über Nacht wird erneut gewogen.

Herstellung der Sprühlösung : 44 mg Taxol werden in 6g Chloroform gelöst.

| Stent Nr. | Vor Beschichtung | Nach Beschichtung | Masse Beschichtung |
|---|---|---|---|
| 1 | 0,0194 g | 0,0197 g | 0,30 mg |

### Beispiel 6

### Ermittlung des Elutionsverhaltens in PBS-Puffer

Je ein Stent wird in einem genügend kleinen Gefäß mit 2 ml PBS-Puffer versetzt, mit Parafilm verschlossen und im Trockenschrank bei 37°C inkubiert. Nach Ablauf der gewählten Zeitintervalle wird jeweils der Überstand abpipettiert und dessen UV-Absorption bei 306 nm gemessen.

## Patentansprüche

1. Medizinprodukt, wobei zumindest ein Teil der Oberfläche des Medizinprodukts direkt oder über mindestens eine dazwischenliegende biostabile und/oder bioabbaubare Schicht mit einer hemokompatiblen Schicht umfassend mindestens eine Verbindung der Formel I 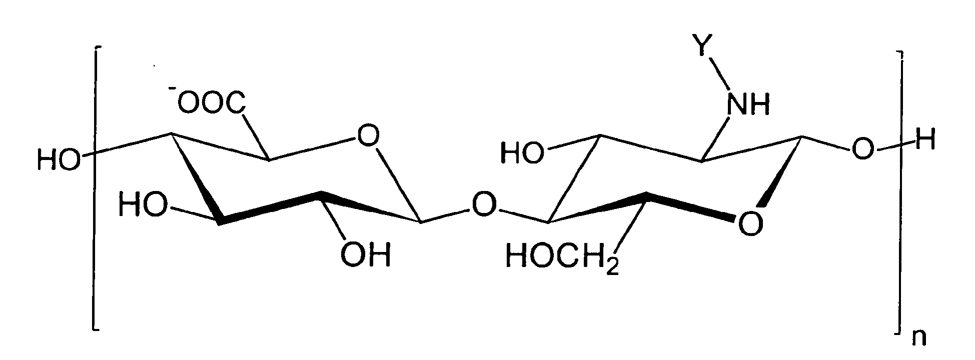 worin
n eine ganze Zahl zwischen 4 und 1050 bedeutet,
Y die Reste -CHO, -COCH₃, -COC₂H₅, -COC₃H₇, -COC₄H₉, -COC₅H₁₁, -COCH(CH₃)₂, -COCH₂CH(CH₃)₂, -COCH(CH₃)C₂H₅, -COC(CH₃)₃, -CH₂COO⁻, -C₂H₄COO⁻, -C₃H₆COO⁻, -C₄H₈COO⁻ darstellt, sowie Salze dieser Verbindungen,
beschichtet ist und auf, in und/oder unter der hemokompatiblen Schicht sich der Wirkstoff. Paclitaxel, befindet.

2. Medizinprodukt gemäß Anspruch 1, worin Y die Reste -CHO, -COCH₃, -COC₂H₅, -COC₃H₇ darstellt, sowie Salze dieser Verbindungen.

3. Medizinprodukt gemäß Anspruch 2, worin Y -COCH₃ ist.

4. Medizinprodukt gemäß einem der vorherigen Ansprüche, wobei sich die hemokompatible Schicht direkt auf der Oberfläche des Medizinprodukts befindet und auf die hemokompatible Schicht Paclitaxel sowie Mischungen dieser Wirkstoffe aufgebracht ist.

5. Medizinprodukt gemäß einem der vorherigen Ansprüche, wobei sich unter der hemokompatiblen Schicht oder zwischen zwei hemokompatiblen Schichten mindestens eine biostabile und/oder bioabbaubare Schicht befindet.

6. Medizinprodukt gemäß einem der vorherigen Ansprüche, wobei die hemokompatible Schicht mit mindestens einer weiteren, darüberliegenden biostabilen und/oder bioabbaubaren Schicht vollständig oder/und unvollständig überzogen ist.

7. Medizinprodukt gemäß einem der vorherigen Ansprüche, wobei mindestens eine Wirkstoffschicht aus Paclitaxel zwischen der biostabilen und der hemokompatiblen Schicht liegt.

8. Medizinprodukt gemäß einem der vorherigen Ansprüche, wobei Paclitaxel kovalent und/oder adhäsiv in und/oder auf der hemokompatiblen Schicht und/oder der biostabilen und/oder der bioabbaubaren Schicht gebunden ist.

9. Medizinprodukt gemäß einem der vorherigen Ansprüche, **dadurch gekennzeichnet, dass** als bioabbaubare Substanzen für die bioabbaubare Schicht Polyvalerolactone, Poly-ε-Decalactone, Polylactonsäure, Polyglycolsäure Polylactide, Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride wie Polymaleinsäure-anhydride, Polyhydroxymethacrylate, Fibrin, Polycyanoacrylate, Polycaprolactondimethylacrylate, Poly-b-Maleinsäure Polycaprolactonbutyl-acrylate, Multiblockpolymere wie z.B. aus Oligocaprolactondiole und Oligodioxanondiole, Polyetherestermultiblockpolymere wie z.B. PEG und Poly(butylenterephtalat. Polypivotolactone, Polyglycolsäuretrimethyl-carbonate Polycaprolacton-glycolide, Poly(g-ethylglutamat), Poly(DTH-Iminocarbonat), Poly(DTE-co-DT-carbonat), Poly(Bisphenol A-iminocarbonat), Polyorthoester, Polyglycol-säuretrimethyl-carbonate, Polytrimethylcarbonate Polyiminocarbonate, Poly(N-vinyl)-Pyrrolidon, Polyvinylalkohole, Polyesteramide, glycolierte Polyester, Polyphosphoester, Polyphosphazene, Poly[p-carboxyphenoxy)propan] Polyhydroxypentan-säure, Polyanhydride, Polyethylenoxid-propylenoxid, weiche Polyurethane, Polyurethane mit Aminosäurereste im Backbone, Polyetherester wie das Polyethylenoxid, Polyalkenoxalate, Polyorthoester sowie deren Copolymere, Lipide, Carrageenane, Fibrinogen, Stärke, Kollagen, protein-basierende Polymere, Polyaminosäuren, synthetische Polyaminosäuren, Zein, modifiziertes Zein, Polyhydroxyalkanoate, Pectinsäure, Actinsäure, modifiziertes und unmodifiziertes Fibrin und Casein, Carboxymethylsulfat, Albumin, desweiteren Hyaluronsäure, Chitosan und seine Derivate, Heparansulfate uns eine Derivate, Heparine, Chondroitinsulfat, Dextran, b-Cyclodextrine, und Copolymere mit PEG und Polypropylenglycol, Gummi arabicum, Guar, Gelatine, Collagen Collagen-N-Hydroxysuccinimid, Lipide, Phospholipide, Modifikationen und Copolymere und/oder Mischungen der vorgenannten Substanzen eingesetzt werden.

10. Medizinprodukt gemäß einem der vorherigen Ansprüche, **dadurch gekennzeichnet, dass** als biostabile Substanzen für die biostabile Schicht Polyacrylsäure und Polyacrylate wie Polymethylmethacrylat, Polybutylmethacrylat, Polyacrylamid, Polyacrylonitrile, Polyamide, Polyetheramide, Polyethylenamin, Polyimide, Polycarbonate, Polycarbourethane, Polyvinylketone, Polyvinylhalogenide, Polyvinylidenhalogenide, Polyvinylether, Polyisobutylene, Polyvinylaromaten, Polyvinylester, Polyvinylpyrollidone, Polyoxymethylene, Polytetramethylenoxid, Polyethylen, Polypropylen, Polytetrafluorethylen, Polyurethane, Polyetherurethane Silicon-Polyetherurethane, Silicon-Polyurethane, Silicon-Polycarbonat-Urethane, Polyolefin-Elastomere, Polyisobutylene, EPDM-Gummis, Fluorosilicone, Carboxymethylchitosane, Polyaryletheretherketone, Polyetheretherketone, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseacetatbutyrate, Ethylvinylacetat-copolymere, Polysulfone, Epoxyharze, ABS-Harze, EPDM-Gummis, Silicone wie Polysiloxane, Polydimethylsiloxane, Polyvinylhalogene und Copolymere, Celluloseether, Cellulosetriacetate. Chitosane und Copolymere und/oder Mischungen derselben eingesetzt werden

11. Medizinprodukt gemäß einem der vorherigen Ansprüche, wobei anstelle des Wirkstoffs Paclitaxel einer der folgenden Wirkstoffe verwendet wird: Simvastatin, Tialin-Natrium, Macrocyclisches Suboxid (MCS), Derivate von MCS, aktiviertes Protein C (aPC), PETN, Trapidil, β-Estradiol sowie Mischungen dieser Wirkstoffe oder Mischungen eines dieser Wirkstoffe mit Paclitaxel.

12. Medizinprodukt gemäß einem der vorherigen Ansprüche, **dadurch gekennzeichnet, dass** es sich bei dem Medizinprodukt um Prothesen, Organe, Gefäße, Aorten, Herzklappen, Schläuchen, Organersatzteilen, Implantaten, Fasern, Hohlfasern, Stents, Kanülen, Spritzen, Membranen, Konserven, Blutbehältern, Titerplatten, Herzschrittmachern, Adsorbermedien, Chromatographiemedien, Chromatographiesäulen, Dialysatoren, Verbindungsstücke, Sensoren, Ventile, Zentrifugenkammern, Wäremaustauschern, Endoskope, Filter, Pumpenkammern handelt.

13. Medizinprodukte nach Anspruch 12, **dadurch gekennzeichnet, dass** es sich bei dem Medizinprodukt um einen Stent handelt.

14. Stents gemäß Anspruch 13, wobei die aufgetragenen Polymermengen zwischen 0,01 mg bis 3 mg / Schicht, bevorzugt 0,20 mg bis 1 mg und insbesondere bevorzugt zwischen 0,2 mg bis 0,5 mg / Schicht liegen.

15. Stent gemäß Anspruch 13 oder 14, **dadurch gekennzeichnet, dass** der Wirkstoff in einer pharmazeutisch aktiven Konzentration von 0,001 - 10 mg pro cm² Stentoberfläche und pro Schicht eingesetzt wird.

16. Stent gemäß eines der Ansprüche 13 - 15 zur Verhinderung oder Reduzierung der Restenose.

17. Stent gemäß eines der Ansprüche 13 - 15 zur kontinuierlichen Freisetzung von Paclitaxel, Simvastatin, Tialin-Natrium, Macrocyclisches Suboxid (MCS), Derivate von MCS, aktiviertes Protein C (aPC), PETN, Trapidil und/oder β-Estradiol.

18. Medizinprodukte nach einem der Ansprüche 1-13 für den direkten Kontakt mit Blut.

19. Verwendung der Medizinprodukte nach einem der Ansprüche 1-13 zur Verhinderung oder Verringerung der unspezifischen Anhaftung und/oder Ablagerung von Proteinen auf den beschichteten Oberflächen der Medizinprodukte.

20. Verwendung nach Anspruch 18 oder 19, **dadurch gekennzeichnet, daß** die hemokompatibel beschichtete Oberfläche des Medizinprodukts die Oberfläche von Mikrotiterplatten oder anderen Trägermedien für diagnostische Nachweisverfahren ist.

21. Verwendung nach Anspruch 18 oder 19, **dadurch gekennzeichnet, daß** die hemokompatibel beschichtete Oberfläche des Medizinprodukts die Oberfläche von Adsorbermedien oder Chromatographiemedien ist.

22. Verfahren zur hemokompatiblen Beschichtung von biologischen und/oder künstlichen Oberflächen von Medizinprodukten umfassend die folgenden Schritte:
a) Bereitstellen einer Oberfläche eines Medizinprodukts und
b) Aufbringen mindestens einer Verbindung der allgemeinen Formel I gemäß Anspruch 1 als hemokompatible Schicht auf diese Oberfläche und/oder
b') Aufbringen einer biostabilen und/oder bioabbaubaren Schicht auf die Oberfläche des Medizinprodukts oder der hemokompatiblen Schicht.

23. Verfahren nach Anspruch 22, wobei die hemokompatible Schicht oder die biostabile und/oder bioabbaubaren Schicht im Tauch- oder Sprühverfahren mit mindestens einer bioabbaubaren und/oder biostabilen Schicht, welche Paclitaxel kovalent und/oder adhäsiv gebunden enthält, überzogen wird.

24. Verfahren nach Anspruch 22 oder 23 umfassend den weiteren Schritt c):
c. Aufbringen von Paclitaxel in und/oder auf die hemokompatible Schicht oder die biostabile und/oder bioabbaubare Schicht.

25. Verfahren nach Anspruch 24, wobei Paclitaxel im Tauch- oder Sprühverfahren auf und/oder in die hemokompatible Schicht oder die biostabile und/oder bioabbaubare Schicht auf- und/oder eingebracht wird und/oder durch kovalente und/oder adhäsive Kopplung an die hemokompatible Schicht oder die biostabile und/oder bioabbaubare Schicht gebunden wird.

26. Verfahren nach einem der Ansprüche 22 - 25, umfassend den weiteren Schritt d):
d. Aufbringen mindestens einer bioabbaubaren Schicht und/oder mindestens einer biostabilen und/oder bioabbaubaren Schicht auf die hemokompatible Schicht bzw. die Schicht aus Paclitaxel,
oder
d') Aufbringen mindestens einer Verbindung der allgemeinen Formel I gemäß Anspruch 1 als hemokompatible Schicht auf die biostabile und/oder bioabbaubaren Schicht oder der Schicht aus Paclitaxel.

27. Verfahren nach einem der Ansprüche 22 - 26, umfassend den weiteren Schritt e.):
e. Aufbringen von Paclitaxel in und/oder auf die mindestens eine bioabbaubare und/oder biostabile Schicht oder die hemokompatible Schicht.

28. Verfahren nach Anspruch 27, wobei Paclitaxel im Tauch- oder Sprühverfahren auf und/oder in die mindestens eine bioabbaubare und/oder biostabile Schicht oder die hemokompatible Schicht auf- und/oder eingebracht wird und/oder durch kovalente und/oder adhäsive Kopplung an die mindestens eine bioabbaubare und/oder biostabile Schicht oder hemokompatible Schicht gebunden wird.

29. Verfahren nach einem der Ansprüche 22 - 28, wobei die biostabile und/oder bioabbaubare Schicht kovalent und/oder adhäsiv an die Oberfläche des Medizinprodukts gebunden wird und die hemokompatible Schicht kovalent an die biostabile und/oder bioabbaubare Schicht gebunden wird und diese vollständig oder unvollständig überzieht.

30. Verfahren nach einem der Ansprüche 22 - 29, **dadurch gekennzeichnet, daß** die hemokompatible Schicht aus Heparin nativen Ursprungs regioselektiv hergestellter Derivate unterschiedlicher Sulfatierungsgrade und Acylierungsgrade im Molekulargewichtsbereich des für die antithrombotische Wirkung verantwortlichen Pentasaccharides bis zum Standardmolekulargewicht des käuflichen Heparins von ca. 13kD, Heparansulfate und seine Derivate, Oligo- und Polysaccharide der Erythrocytenglycocalix, desulfatiertes und N-reacetyliertes Heparin, N-carboxymethyliertes und/oder partiell N-acetyliertes Chitosan sowie aus Mischungen dieser Substanzen besteht.

31. Verfahren nach einem der Ansprüche 22 - 30, **dadurch gekennzeichnet, dass** als bioabbaubare Substanzen für die bioabbaubare Schicht Polyvalerolactone, Poly-ε-Decalactone, Polylactonsäure, Polyglycolsäure Polylactide, Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride wie Polymaleinsäure-anhydride, Polyhydroxymethacrylate, Fibrin, Polycyanoacrylate, Polycaprolactondimethylacrylate, Poly-b-Maleinsäure Polycaprolactonbutyl-acrylate, Multiblockpolymere wie z.B. aus Oligocaprolactondiole und Oligodioxanondiole, Polyetherestermultiblockpolymere wie z.B. PEG und Poly(butylenterephtalat. Polypivotolactone, Polyglycolsäuretrimethyl-carbonate Polycaprolacton-glycolide, Poly(g-ethylglutamat), Poly(DTH-Iminocarbonat), Poly(DTE-co-DT-carbonat), Poly(Bisphenol A-iminocarbonat), Polyorthoester, Polyglycolsäuretrimethyl-carbonate, Polytrimethylcarbonate Polyiminocarbonate, Poly(N-vinyl)-Pyrrolidon, Polyvinylalkohole, Polyesteramide, glycolierte Polyester, Polyphosphoester, Polyphosphazene, Poly[p-carboxyphenoxy)propan] Polyhydroxypentan-säure, Polyanhydride, Polyethylenoxid-propylenoxid, weiche Polyurethane, Polyurethane mit Aminosäurereste im Backbone, Polyetherester wie das Polyethylenoxid, Polyalkenoxalate, Polyorthoester sowie deren Copolymere, Lipide, Carrageenane, Fibrinogen, Stärke, Kollagen, protein-basierende Polymere, Polyaminosäuren, synthetische Polyaminosäuren, Zein, modifiziertes Zein, Polyhydroxyalkanoate, Pectinsäure, Actinsäure, modifiziertes und unmodifiziertes Fibrin und Casein, Carboxymethylsulfat, Albumin, desweiteren Hyaluronsäure, Chitosan und seine Derivate, Heparansulfate uns eine Derivate, Heparine, Chondroitinsulfat, Dextran, b-Cyclodextrine, und Copolymere mit PEG und Polypropylenglycol, Gummi arabicum, Guar, Gelatine, Collagen Collagen-N-Hydroxysuccinimid, Lipide, Phospholipide, Modifikationen und Copolymere und/oder Mischungen der vorgenannten Substanzen eingesetzt werden.

32. Verfahren nach einem der Ansprüche 22 - 31, **dadurch gekennzeichnet, dass** als biostabile Substanzen für die biostabile Schicht Polyacrylsäure und Polyacrylate wie Polymethylmethacrylat, Polybutylmethacrylat, Polyacrylamid, Polyacrylonitrile, Polyamide, Polyetheramide, Polyethylenamin, Polyimide, Polycarbonate, Polycarbourethane, Polyvinylketone, Polyvinylhalogenide, Polyvinylidenhalogenide, Polyvinylether, Polyisobutylene, Polyvinylaromaten, Polyvinylester, Polyvinylpyrollidone, Polyoxymethylene, Polytetramethylenoxid, Polyethylen, Polypropylen, Polytetrafluorethylen, Polyurethane, Polyetherurethane Silicon-Polyetherurethane, Silicon-Polyurethane, Silicon-Polycarbonat-Urethane, Polyolefin-Elastomere, Polyisobutylene, EPDM-Gummis, Fluorosilicone, Carboxymethylchitosane, Polyaryletheretherketone, Polyetheretherketone, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseacetatbutyrate, Ethylvinylacetat-copolymere, Polysulfone, Epoxyharze, ABS-Harze, EPDM-Gummis, Silicone wie Polysiloxane, Polydimethylsiloxane, Polyvinylhalogene und Copolymere, Celluloseether, Cellulosetriacetate. Chitosane und Copolymere und/oder Mischungen derselben eingesetzt werden.

33. Verfahren nach einem der Ansprüche 22 - 32, **dadurch gekennzeichnet, daß** die Aufbringung Polysaccharide der Formel I gemäß Anspruch 1 durch hydrophobe Wechselwirkungen, van der Waals Kräfte, elektrostatische Wechselwirkungen, Wasserstoffbrücken, ionische Wechselwirkungen, Quervernetzung und/oder kovalente Bindung erfolgt.

34. Verfahren nach einem der Ansprüche 22 - 33, wobei anstelle des Wirkstoffs Paclitaxel einer der folgenden Wirkstoffe verwendet wird: Simvastatin, Tialin-Natrium, Macrocyclisches Suboxid (MCS), Derivate von MCS, aktiviertes Protein C (aPC), PETN, Trapidil, β-Estradiol sowie Mischungen dieser Wirkstoffe oder Mischungen eines dieser Wirkstoffe mit Paclitaxel.

35. Medizinprodukt erhältlich nach einem Verfahren gemäß eines des Ansprüche 22 - 34.

## Claims

1. Medical device, wherein at least one part of the surface of the medical device is coated directly or via at least one intervening biostable and/or biodegradable layer with a hemocompatible layer comprising at least one compound of the formula 1 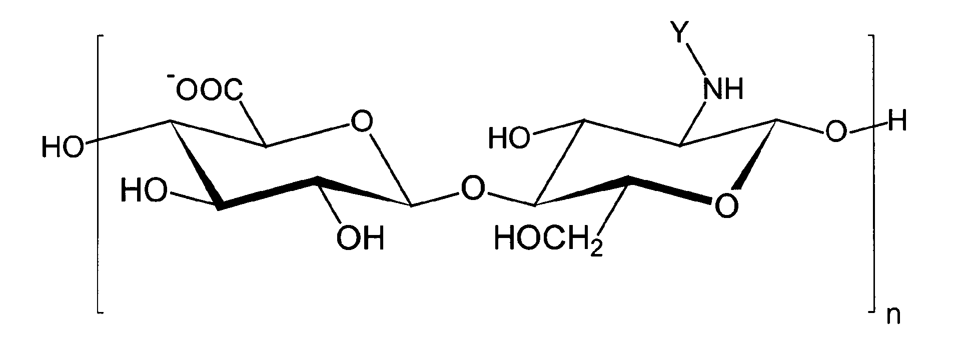 wherein
n is an integer between 4 and 1050,
Y represents the residues -CHO, -COCH₃, -COC₂H₅, -COC₃H₇, -COC₄H₉, -COC₅H₁₁, -COCH(CH₃)₂, -COCH₂CH(CH₃)₂, -COCH(CH₃)C₂H₅, -COC(CH₃)₃, -CH₂COO⁻, -C₂H₄COO⁻, -C₃H₆COO⁻, -C₄H₈COO⁻,
as well as salts of said compounds,
and the active agent paclitaxel is present on, in and/or under the hemocompatible layer.

2. Medical device according to claim 1, wherein Y represents the residues -CHO, -COCH₃, -COC₂H₅, -COC₃H₇, as well as salts of these compounds.

3. Medical device according to claim 2, wherein Y is -COCH₃.

4. Medical device according to one of the preceding claims, wherein the hemocompatible layer is directly placed on the surface of the medical device and onto said hemocompatible layer paclitaxel as well as mixtures of these active agents are deposited.

5. Medical device according to any one of the preceding claims, wherein at least one biostable and/or biodegradable layer is present under the hemocompatible layer or between two hemocompatible layers.

6. Medical device according to any one of the preceding claims, wherein the hemocompatible layer is coated completely and/or incompletely with at least one additional biostable and/or biodegradable layer situated above.

7. Medical device according to any one of the preceding claims, wherein at least one active agent layer of paclitaxel is present between the biostable and the hemocompatible layer.

8. Medical device according to any one of the preceding claims, wherein paclitaxel is bound covalently and/or adhesively in and/or on the hemocompatible layer and/or the biostable and/or the biodegradable layer.

9. Medical device according to any one of the preceding claims, **characterized in that** polyvalerolactones, poly-ε-decalactones, polylactonic acid, polyglycolic acid, polylactides, polyglycolides, copolymers of the polylactides and polyglycolides, poly-ε-caprolactone, polyhydroxybutyric acid, polyhydroxybutyrates, polyhydroxyvalerates, polyhydroxybutyrate-co-valerates, poly(1,4-dioxane-2,3-one), poly(1,3-dioxane-2-one), poly-para-dioxanone, polyanhydrides such as polymaleic acid anhydrides, polyhydroxy methacrylates, fibrin, polycyanoacrylates, polycaprolactone dimethylacrylates, poly-b-maleic acid, polycaprolactone butyl acrylates, multiblock polymers such as from oligocaprolactonediols and oligodioxanonediols, polyetherester multiblock polymers such as PEG and poly(butylene terephtalate), polypivotolactones, polyglycolic acid trimethyl-carbonates, polycaprolactone glycolides, poly(γ-ethyl glutamate), poly(DTH-iminocarbonate), poly(DTE-co-DT-carbonate), poly(bisphenol-A-iminocarbonate), polyorthoesters, polyglycolic acid trimethyl carbonates, polytrimethyl carbonates, polyimino carbonates, poly(N-vinyl)-pyrrolidone, polyvinyl alcohols, polyesteramides, glycolated polyesters, polyphosphoesters, polyphosphazenes, poly[p-carboxyphenoxy)propane], polyhydroxy pentanoic acid, polyanhydrides, polyethylene oxide propylene oxide, soft polyurethanes, polyurethanes with amino acid residues in the backbone, polyetheresters such as polyethylene oxide, polyalkene oxalates, polyorthoesters as well as their copolymers, lipids, carrageenans, fibrinogen, starch, collagen, protein based polymers, synthetic polyamino acids, modified zein, polyhydroxyalkanoates, pectic acid, actic acid, modified and non modified fibrin and casein, carboxymethyl sulfate, albumin, moreover hyaluronic acid, chitosan and derivatives thereof, heparan sulfates and derivatives thereof, heparins, chondroitin sulfate, dextran, b-cyclodextrins, and copolymers with PEG and polypropylene glycol, gum arabic guar, gelatine, collagen-N-hydroxysuccinimide, phospholipids, modifications and copolymers and/or mixtures of the aforementioned substances can be used as biodegradable substances for the biodegradable layer.

10. Medical device according to any one of the preceding claims, **characterized in that** polyacrylic acid and polyacrylates such as polymethyl methacrylate, polybutyl methacrylate, polyacrylamide, polyacrylonitriles, polyamides, polyetheramides, polyethylene amine, polyimides, polycarbonates, polycarbourethanes, polyvinyl ketones, polyvinyl halogenides, polyvinylidene halogenides, polyvinyl ethers, polyisobutylenes, polyvinyl aromatic compounds, polyvinyl esters, polyvinyl pyrrolidones, polyoxymethylenes, polytetramethylene oxide, polyethylene, polypropylene, polytetrafluoroethylene, polyurethanes, polyether urethanes, silicone polyether urethanes, silicone polyurethanes, silicone polycarbonate urethanes, polyolefin elastomers, EPDM gums, fluorosilicones, carboxymethyl chitosans, polyaryletherether ketones, polyetherether ketones, polyethylene terephthalate, polyvalerates, carboxymethylcellulose, cellulose, rayon, rayon triacetates, cellulose nitrates, cellulose acetates, hydroxyethyl cellulose, cellulose butyrates, cellulose acetate butyrates, ethyl vinyl acetate copolymers, polysulfones, epoxy resins, ABS resins, silicones such as polysiloxanes, polydimethylsiloxanes, polyvinyl halogens and copolymers, cellulose ethers, cellulose triacetates, chitosans and copolymers and/ or mixtures of these substances are used as biostable substances for the biostable layer.

11. Medical device according to any one of the preceding claims, wherein one of the following active agents is used instead of the active agent paclitaxel: simvastatin, thialin-sodium, macrocyclic suboxide (MCS), derivatives of MCS, activated protein C (aPC), PETN, trapidil, β-estradiol as well as mixtures of these active agents or mixtures of one of these active agents with paclitaxel.

12. Medical device according to any one of the preceding claims, **characterized in that** the medical device comprises prostheses, organs, vessels, aortas, heart valves, tubes, organ spare parts, implants, fibres, hollow fibres, stents, hollow needles, syringes, membranes, tinned goods, blood containers, titre plates, pacemakers, adsorbing media, chromatography media, chromatography columns, dialyzers, connexion parts, sensors, valves, centrifugal chambers, heat exchangers, endoscopes, filters, pump chambers.

13. Medical devices according to claim 12, **characterized in that** the medical device is a stent.

14. Stents according to claim 13, wherein the polymer is deposited in amounts between 0.01 mg to 3 mg/ layer, preferably between 0.20 mg to 1 mg and most preferably between 0.2 mg to 0.5 mg/ layer.

15. Stent according to claim 13 or 14, **characterized in that** the active agent is used in a pharmaceutically active concentration of 0.001 - 10 mg per cm² of stent surface and per layer.

16. Stent according to any one of claims 13 - 15 for the prevention or reduction of restenosis.

17. Stent according to any one of claims 13 - 15 for continuous release of paclitaxel, simvastatin, tialin-sodium, macrocyclic suboxide (MCS), derivatives of MCS, activated protein C (aPC), PETN, trapidil and/or β-estradiol.

18. Medical devices according to any one of claims 1 - 13 for the direct contact with blood.

19. Use of the medical devices according to any one of claims 1 - 13 for the prevention or reduction of unspecific adhesion and/or deposition of proteins on the coated surfaces of the medical devices.

20. Use according to claim 18 or 19, **characterized in that** the hemocompatibly coated surface of the medical device is a surface of microtitre plates or other carrier media for diagnostic detection methods.

21. Use according to claim 18 or 19, **characterized in that** the hemocompatibly coated surface of the medical device is the surface of adsorbing media or chromatography media.

22. Method for the hemocompatible coating of biological and/or artificial surfaces of medical devices comprising the following steps:
a) providing a surface of a medical device and
b) applying at least one compound of the general formula 1 according to claim 1 as hemocompatible layer onto this surface and/or
b') applying a biostable and/or biodegradable layer on the surface of the medical device or the hemocompatible layer.

23. Method according to claim 22, wherein the hemocompatible layer or the biostable and/or biodegradable layer is coated with at least one biodegradable and/or biostabile layer by means of a dipping or spraying method, which layer contains paclitaxel covalently and/or adhesively bound.

24. Method according to claim 22 or 23 comprising the additional step c):
c. applying paclitaxel in and/or on the hemocompatible layer or the biostable and/or biodegradable layer.

25. Method according to claim 24, wherein paclitaxel is applied onto and/or integrated into the hemocompatible layer or the biostable and/or biodegradable layer by means of a dipping or spraying method and/or is bound by covalent and/or adhesive coupling to the hemocompatible layer or the biostable and/or biodegradable layer.

26. Method according to any one of claims 22 - 25, comprising the additional step d) or d'):
d. applying at least one biodegradable layer and/or at least one biostable and/or biodegradable layer on the hemocompatible layer or respectively the layer of paclitaxel,
or
d') applying at least one compound of the general formula 1 according to claim 1 as hemocompatible layer onto the biostable and/or biodegradable layer or the layer of paclitaxel.

27. Method according to any one of claims 22 - 26, comprising the additional step e.):
e. applying paclitaxel into and/or onto the at least one biodegradable and/or biostable layer or the hemocompatible layer.

28. Method according to claim 27, wherein paclitaxel is applied onto and/or integrated into the at least one biodegradable and/or biostable layer or the hemocompatible layer by means of dipping or spraying methods and/or is bound via covalent and/or adhesive coupling to the at least one biodegradable and/or biostable layer or the hemocompatible layer.

29. Method according to any one of claims 22 - 28, wherein the biostable and/or biodegradable layer is covalently and/or adhesively bound to the surface of the medical device and the hemocompatible layer is covalently bound to the biostable and/or biodegradable layer and covers it completely or incompletely.

30. Method according to any one of claims 22 - 29, **characterized in that** the hemocompatible layer comprises heparin of native origin of regioselectively synthesised derivatives of different degrees of sulfatation and degrees of acylation in the range from the molecular weight of the pentasaccharide which is responsible for the antithrombotic activity to the standard molecular weight of the commercially available heparin of about 13 kD, heparan sulfate and derivatives thereof, oligosaccharides and polysaccharides of the glycocalix of erythrocytes, desulfated and N-reacetylated heparin, N-carboxymethylated and/or partially N-acetylated chitosan as well as mixtures of these substances.

31. Method according to any one of claims 22 - 30, **characterized in that** polyvalerolactones, poly-ε-decalactones, polylactonic acid, polyglycolic acid, polylactides, polyglycolides, copolymers of the polylactides and polyglycolides, poly-ε-caprolactone, polyhydroxybutyric acid, polyhydroxybutyrates, polyhydroxyvalerates, polyhydroxybutyrate-co-valerates, poly(1,4-dioxane-2,3-one), poly(1,3-dioxane-2-one), poly-para-dioxanone, polyanhydrides such as polymaleic acid anhydrides, polyhydroxy methacrylates, fibrin, polycyanoacrylates, polycaprolactone dimethylacrylates, poly-b-maleic acid, polycaprolactone butyl acrylates, multiblock polymers such as from oligocaprolactonediols and oligodioxanonediols, polyetherester multiblock polymers such as PEG and poly(butylene terephtalate), polypivotolactones, polyglycolic acid trimethyl-carbonates, polycaprolactone glycolides, poly(γ-ethyl glutamate), poly(DTH-iminocarbonate), poly(DTE-co-DT-carbonate), poly(bisphenol-A-iminocarbonate), polyorthoesters, polyglycolic acid trimethyl carbonates, polytrimethyl carbonates, polyimino carbonates, poly(N-vinyl)-pyrrolidone, polyvinyl alcohols, polyesteramides, glycolated polyesters, polyphosphoesters, polyphosphazenes, poly[p-carboxyphenoxy)propane], polyhydroxy pentanoic acid, polyanhydrides, polyethylene oxide propylene oxide, soft polyurethanes, polyurethanes with amino acid residues in the backbone, polyetheresters such as polyethylene oxide, polyalkene oxalates, polyorthoesters as well as their copolymers, lipids, carrageenans, fibrinogen, starch, collagen, protein based polymers, synthetic polyamino acids, modified zein, polyhydroxyalkanoates, pectic acid, actic acid, modified and non modified fibrin and casein, carboxymethyl sulfate, albumin, moreover hyaluronic acid, chitosan and derivatives thereof, heparan sulfates and derivatives thereof, heparins, chondroitin sulfate, dextran, b-cyclodextrins, and copolymers with PEG and polypropylene glycol, gum arabic guar, gelatine, collagen-N-hydroxysuccinimide, phospholipids, modifications and copolymers and/or mixtures of the aforementioned substances can be used as biodegradable substances for the biodegradable layer.

32. Method according to any one of claims 22 - 31, **characterized in that** polyacrylic acid and polyacrylates such as polymethyl methacrylate, polybutyl methacrylate, polyacrylamide, polyacrylonitriles, polyamides, polyetheramides, polyethylene amine, polyimides, polycarbonates, polycarbourethanes, polyvinyl ketones, polyvinyl halogenides, polyvinylidene halogenides, polyvinyl ethers, polyisobutylenes, polyvinyl aromatic compounds, polyvinyl esters, polyvinyl pyrrolidones, polyoxymethylenes, polytetramethylene oxide, polyethylene, polypropylene, polytetrafluoroethylene, polyurethanes, polyether urethanes, silicone polyether urethanes, silicone polyurethanes, silicone polycarbonate urethanes, polyolefin elastomers, EPDM gums, fluorosilicones, carboxymethyl chitosans, polyaryletherether ketones, polyetherether ketones, polyethylene terephthalate, polyvalerates, carboxymethylcellulose, cellulose, rayon, rayon triacetates, cellulose nitrates, cellulose acetates, hydroxyethyl cellulose, cellulose butyrates, cellulose acetate butyrates, ethyl vinyl acetate copolymers, polysulfones, epoxy resins, ABS resins, silicones such as polysiloxanes, polydimethylsiloxanes, polyvinyl halogens and copolymers, cellulose ethers, cellulose triacetates, chitosans and copolymers and/ or mixtures of these substances are used as biostable substances for the biostable layer.

33. Method according to any one of claims 22 - 32, **characterized in that** the application of the polysaccharides of the formula 1 according to claim 1 is achieved by hydrophobic interactions, van der Waals forces, electrostatic interactions, hydrogen bonds, ionic interactions, cross-linking and/or covalent bonds.

34. Method according to any one of claims 22 - 33, wherein one of the following active agents is used instead of the active agent paclitaxel: simvastatin, tialin-sodium, macrocyclic suboxide (MCS), derivatives of MCS, activated protein C (aPC), PETN, trapidil, β-estradiol as well as mixtures of these active agents or mixtures of one of these active agents with paclitaxel.

35. Medical device which can be obtained according to one method of any one of claims 22 - 34.

## Revendications

1. Dispositif médical, où au moins une partie de la surface du dispositif médical est directement ou par l'intermédiaire d'au moins une couche intermédiaire biologiquement stable et/ ou biodégradable revêtue avec une couche hémocompatible comprenant au moins un composé suivant la formule I 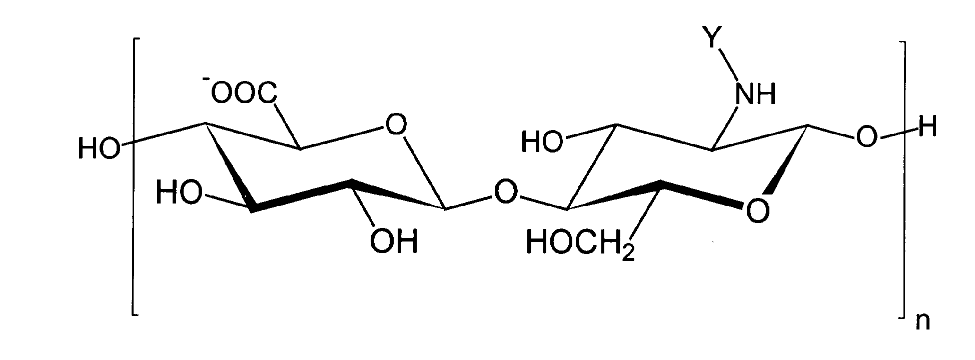 où
n signifie un nombre entier entre 4 et 1050,
Y reprèsente les résidus -CHO, -COCH₃, -COC₂H₅, -COC₃H₇, -COC₄H₉, -COC₅H₁₁, -COCH(CH₃)₂, -COCH₂CH(CH₃)₂, -COCH(CH₃)C₂H₅, -COC(CH₃)₃, -CH₂COO⁻, -C₂H₄COO⁻, -C₃H₆COO⁻, -C₄H₈COO⁻ ainsi que des sels de ces composés,
et l'agent actif Paclitaxel est présent sur, dans et/ ou au-dessous de la couche hémocompatible.

2. Dispositif médical selon la revendication 1, où Y reprèsente les résidus -CHO, -COCH₃, -COC₂H₅, -COC₃H₇ ainsi que des sels de ces composés.

3. Dispositif médical selon la revendication 2, où Y est -COCH₃.

4. Dispositif médical selon l'une quelconque des revendications précédentes, où la couche hémocompatible est située directement sur la surface du dispositif médical et Paclitaxel ainsi que des mélanges de ces agents actifs est/ sont appliqué/es sur la couche hémocompatible.

5. Dispositif médical selon l'une quelconque des revendications précédentes, où au moins une couche biologiquement stable et/ ou biodégradable est située au-dessous de la couche hémocompatible ou entre deux couches hémocompatibles.

6. Dispositif médical selon l'une quelconque des revendications précédentes, où la couche hémocompatible est recouverte, complètement ou incomplètement, avec au moins une autre couche biologiquement stable et/ ou biodégradable sus-jacente.

7. Dispositif médical selon l'une quelconque des revendications précédentes, où au moins une couche d'agent actif composée de Paclitaxel est située entre la couche biologiquement stable et la couche hémocompatible.

8. Dispositif médical selon l'une quelconque des revendications précédentes, où Paclitaxel est lié de manière covalente et/ ou adhésive dans et/ ou sur la couche hémocompatible et/ ou la couche biologiquement stable et/ ou la couche biodégradable.

9. Dispositif médical selon l'une quelconque des revendications précédentes, **caractérisé en ce que** les substances suivantes sont utilisées comme substances biodégradables pour la couche biodégradable: polyvalérolactones, poly-ε-décalactones, polyacide lactonique, acide polyglycolique, polylactides, polyglycolides, copolymères des polylactides et polyglycolides, poly-ε-caprolactone, polyhydroxy acide butyrique, polyhydroxybutyrates, polyhydroxyvalérates, polyhydroxybutyrates-co-valérates, poly(1,4-dioxane-2,3-dione), poly(1,3-dioxane-2-dione), poly-para-dioxanone, polyanhydrides comme des anhydrides de l'acide polymaléique, polyhydroxyméthacrylates, fibrine, polycyanoacrylates, polycaprolactone diméthylacrylates, poly-β-acide maléique, polycaprolactone butyl acrylates, polymères séquencés multiples comme p.ex. d'oligocaprolactone diols et oligodioxanone diols, polymères séquencés multiples de polyéthers comme p.ex. PEG et poly(butylène téréphtalate), polypivotolactones, triméthyl carbonates de l'acide polyglycolique, polycaprolactone-glycolides, poly(γ-éthylglutamate), poly(DTH-imino-carbonate), poly(DTE-co-DT-carbonate), poly(bisphenol A-imino-carbonate), polyorthoesters, poly(triméthyl-carbonates), poly(imino-carbonates), poly(N-vinyle)-pyrrolidone, alcools polyvinyliques, polyesteramides, polyesters glycolés, poly(phosphoesters), polyphosphazènes, poly[p-carboxyphénoxy)propane], polyhydroxy-acide valérique, polyanhydrides, oxyde de polyéthylène-oxyde de propylène, polyuréthanes souples, polyuréthanes ayant des résidus d'acide aminé dans la chaîne principale, polyétheresters comme l'oxyde de polyéthylène, poly(alcène oxalate) polyorthoesters ainsi que leurs copolymères, lipides, carraghénanes, fibrinogène, amidon, collagène, polymères à base de protéines, acides polyaminés, acides polyaminés synthétiques, zéine, zéine modifiée, polyhydroxyalcanoates, acide pectique, acide actique, fibrine et caséine modifiées et non modifiées, sulfate de carboxyméthyl, albumine, en outre acide hyaluronique, chitosane et ses dérivés, sulfates d'héparane et leurs dérivés, héparines, sulfate de chondroïtine, dextrane, β-cyclodextrines et copolymères avec PEG et polypropylène glycol, gomme arabique, guar, gelatine, collagène-N-hydroxy succinimide, phospholipides, modifications et copolymères et/ ou mélanges des substances susmentionnées.

10. Dispositif médical selon l'une quelconque des revendications précédentes, **caractérisé en ce que** les substances suivantes sont utilisées comme substances biologiquement stables pour la couche biologiquement stable : acide polyacrylique et polyacrylates ainsi que polyméthylméthacrylate, polybutylméthacrylate, polyacrylamide, polyacrylonitriles, polyamides, polyétheramides, polyéthylène amine, polyimides, polycarbonates, polycarbo-uréthane, cétones de polyvinyle, halogénides de polyvinyle, halogénides de polyvinylidène, éthers de polyvinyle, polyisobutylènes, aromatiques polyvinyliques, esters polyvinyliques, pyrrolidones de polyvinyle, polyoxymethylène, oxyde de polytétraméthylène, polyéthylène, polypropylène, polytétrafluoroéthylène, polyuréthanes, polyétheruréthanes, polyétheruréthanes de silicone, polyuréthanes de silicone, polycarbonateuréthanes de silicone, élastomères de polyoléfine, gommes EPDM, fluorosilicones, chitosanes de carboxyméthyl, poly(aryléther éther cétones), poly(éther éther cétones), poly(éthylène téréphtalate), polyvalérates, cellulose de carboxyméthyl, cellulose, viscose, triacétates de viscose, nitrates de cellulose, acétates de cellulose, cellulose de hydroxyéthyle, butyrates de cellulose, acetate-butyrates de cellulose, copolymères d'éthylevinyle acétate, polysulfones, résines époxy, résines ABS, silicones comme les polysiloxanes, polydiméthyl siloxanes, halogènes de polyvinyle et copolymères, éthers de cellulose, triacétates de cellulose, chitosane et copolymères et/ ou mélanges de ceux-ci.

11. Dispositif médical selon l'une quelconque des revendications précédentes, où au lieu de l'agent actif Paclitaxel un des agents actifs suivants est utilisé : simvastatine, sodium de tialine, sous-oxyde macrocyclique (MCS), dérivés de MCS, protéine C activée (aPC), PETN, trapidil, β-estradiol ainsi que des mélanges de ces agents actifs ou des mélanges d'un de ces agents actifs avec Paclitaxel.

12. Dispositif médical selon l'une quelconque des revendications précédentes, **caractérisé en ce que** le dispositif médical est une prothèse, un organe, un vaisseau, un aorte, une valve cardiaque, un tuyau, une greffe d'organe, un implant, une fibre, une fibre creuse, un stent, une canule, une seringue, une membrane, un flacon de sang conservé, un récipient de sang, une plaque de titration, un stimulateur cardiaque, un médium d'adsorption, un médium de chromatographie, une colonne de chromatographie, un dialyseur, un élément de raccord, un capteur, une soupape, une chambre de centrifugation, un échangeur de chaleur, un endoscope, un filtre, une chambre de pompage.

13. Dispositifs médicaux selon la revendication 12, **caractérisé en ce que** le dispositif médical est un stent.

14. Stents selon la revendication 13, où les quantités de polymères appliquées sont entre 0,01 mg à 3 mg/couche, de préférence 0,20 mg à 1 mg et particulièrement préféré entre 0,2 mg à 0,5 mg/couche.

15. Stent selon la revendication 13 ou 14, **caractérisé en ce que** l'agent actif est utilisé dans une concentration pharmaceutiquement active de 0,001 - 10 mg par cm² surface de stent et par couche.

16. Stent selon l'une quelconque des revendications 13 - 15 destiné à la prévention ou la réduction de la resténose.

17. Stent selon l'une des revendications 13 - 15 destiné à la liberation continue de Paclitaxel, simvastatine, sodium de tialine, sous-oxyde macrocyclique (MCS), dérivés de MCS, protéine C activée (aPC), PETN, trapidil et/ ou β-estradiol.

18. Dispositifs médicaux selon l'une des révendications 1 - 13 destinés au contact direct avec le sang.

19. Utlilisation des dispositifs médicaux selon l'une des révendications 1 - 13 destinée à la prévention ou la réduction de l'adhésion non spécifique et/ ou du dépôt de protéines sur les surfaces revêtues des dispositifs médicaux.

20. Utilisation selon la revendication 18 ou 19, **caractérisée en ce que** la surface du dispositif médical revêtue de manière hémocompatible est la surface de microplaques de tritration ou d'autres médias de support pour des procédés de detection diagnostiques.

21. Utilisation selon la revendication 18 ou 19, **caractérisée en ce que** la surface du dispositif médical revêtue de manière hémocompatible est la surface de médias d'absorption ou de médias de chromatographie.

22. Procédé destiné au revêtement hémocompatible de surfaces biologiques et/ ou artificielles de dispositifs médicaux comprenant les étapes suivantes :
a.) mettre à disposition une surface d'un dispositif medical et
b.) appliquer au moins un composé selon la formule générale I selon la revendication 1 comme couche hémocompatible sur cette surface et/ ou
b') appliquer une couche biologiquement stable et/ ou biodégradable sur la surface du dispositif médical ou la couche hémocompatible.

23. Procédé selon la revendication 22, où la couche hémocompatible ou la couche biologiquement stable et/ ou biodégradable est recouverte, dans le cadre d'un procédé d'immersion ou de pulvérisation, avec au moins une couche biodégradable et/ ou biologiquement stable qui contient Paclitaxel lié de manière covalente et/ ou adhésive.

24. Procédé selon la revendication 22 ou 23 comprenant l'autre étape c) :
c.) appliquer Paclitaxel dans et/ ou sur la couche hémocompatible ou la couche biologiquement stable et/ ou biodégradable.

25. Procédé selon la revendication 24, où Paclitaxel est appliqué et/ ou introduit sur et/ ou dans la couche hémocompatible ou la couche biologiquement stable et/ ou biodégradable par procédé d'immersion ou de pulvérisation et/ ou Paclitaxel est lié par liaison covalente et/ ou adhésive à la couche hémocompatible ou la couche biologiquement stable et/ ou biodégradable.

26. Procédé selon l'une des revendications 22 - 25, comprenant l'autre étape d)
ou d') :
d.) appliquer au moins une couche biodégradable et/ ou au moins une couche biologiquement stable et/ ou biodégradable sur la couche hémocompatible respectivement la couche composée de Paclitaxel,
ou
d') appliquer au moins un composé selon la formule générale I selon la revendication 1 comme couche hémocompatible sur la couche biologiquement stable et/ ou biodégradable ou la couche composée de Paclitaxel.

27. Procédé selon l'une des revendications 22 - 26 comprenant l'autre étape e.):
e.) appliquer Paclitaxel dans et/ ou sur la au moins une couche biodégradable et/ ou biologiquement stable ou la couche hémocompatible.

28. Procédé selon la revendication 27, où Paclitaxel est appliqué et/ ou introduit sur et/ ou dans la au moins une couche biodégradable et/ ou biologiquement stable ou la couche hémocompatible par procédé d'immersion ou de pulvérisation et/ ou Paclitaxel est lié par liaison covalente et/ ou adhésive à la au moins une couche biodégradable et/ ou biologiquement stable ou la couche hémocompatible.

29. Procédé selon l'une des revendications 22 - 28, où la couche biologiquement stable et/ ou biodégradable est liée de manière covalente et/ ou adhésive à la surface du dispositif médical et où la couche hémocompatible est liée de manière covalente à la couche biologiquement stable et/ ou biodégradable et recouvre celle-ci complètement ou incomplètement.

30. Procédé selon l'une des revendications 22 - 29, **caractérisé en ce que** la couche hémocompatible se compose de dérivés produits de manière regioséléctive à partir d'héparine d'origine native ayant différents degrés de sulfatation et d'acylation dans la plage du poids moléculaire allant du pentasaccharide responsable de l'effet antithrombotique jusqu'au poids moléculaire standard de l'héparine commerciale d'environ 13 kD ; sulfates d'héparane et leurs dérivés, oligo- et polysaccharides de la glycocalyx de l'érythrocyte, héparine désulfatée et N-reacetylée, chitosane N-carboxymethylé et/ ou partiellement N-acetylé ainsi que des mélanges de ces substances.

31. Procédé selon l'une des revendications 22 - 30, **caractérisé en ce que** les substances suivantes sont utilisées comme substances biodégradables pour la couche biodégradable : polyvalérolactones, poly-ε-décalactones, polyacide lactonique, acide polyglycolique, polylactides, polyglycolides, copolymères des polylactides et polyglycolides, poly-ε-caprolactone, polyhydroxy acide butyrique, polyhydroxybutyrates, polyhydroxyvalérates, polyhydroxybutyrates-co-valérates, poly(1,4-dioxane-2,3-dione), poly(1,3-dioxane-2-dione), poly-para-dioxanone, polyanhydrides comme des anhydrides de l'acide polymaléique, polyhydroxyméthacrylates, fibrine, polycyanoacrylates, polycaprolactone diméthylacrylates, poly-β-acide maléique, polycaprolactone butyl acrylates, polymères séquencés multiples comme p.ex. d'oligocaprolactone diols et oligodioxanone diols, polymères séquencés multiples de polyéthers comme p.ex. PEG et poly(butylène téréphtalate), polypivotolactones, triméthyl carbonates de l'acide polyglycolique, polycaprolactone-glycolides, poly(γ-éthylglutamate), poly(DTH-imino-carbonate), poly(DTE-co-DT-carbonate), poly(bisphenol A-imino-carbonate), polyorthoesters, poly(triméthyl-carbonates), poly(iminocarbonates), poly(N-vinyle)-pyrrolidone, alcools polyvinyliques, polyesteramides, polyesters glycolés, poly(phosphoesters), polyphosphazènes, poly[p-carboxyphénoxy)propane], polyhydroxy-acide valérique, polyanhydrides, oxyde de polyéthylène-oxyde de propylène, polyuréthanes souples, polyuréthanes ayant des résidus d'acide aminé dans la chaîne principale, polyétheresters comme l'oxyde de polyéthylène, poly(alcène oxalate) polyorthoesters ainsi que leurs copolymères, lipides, carraghénanes, fibrinogène, amidon, collagène, polymères à base de protéines, acides polyaminés, acides polyaminés synthétiques, zéine, zéine modifiée, polyhydroxyalcanoates, acide pectique, acide actique, fibrine et caséine modifiées et non modifiées, sulfate de carboxyméthyl, albumine, en outre acide hyaluronique, chitosane et ses dérivés, sulfates d'héparane et leurs dérivés, héparines, sulfate de chondroïtine, dextrane, β-cyclodextrines et copolymères avec PEG et polypropylène glycol, gomme arabique, guar, gelatine, collagène-N-hydroxy succinimide, phospholipides, modifications et copolymères et/ ou mélanges des substances susmentionnées.

32. Procédé selon l'une des revendications 22 - 31, **caractérisé en ce que** les substances suivantes sont utilisées comme substances biologiquement stables pour la couche biologiquement stable : acide polyacrylique et polyacrylates comme polyméthylméthacrylate, polybutylméthacrylate, polyacrylamide, polyacrylonitriles, polyamides, polyétheramides, polyéthylène amine, polyimides, polycarbonates, polycarbo-uréthanes, cétones de polyvinyle, halogénides de polyvinyle, halogénides de polyvinylidène, éthers de polyvinyle, polyisobutylènes, aromatiques polyvinyliques, polyvinyl ésters, pyrrolidones de polyvinyle, polyoxymethylène, oxyde de polytétraméthylène, polyéthylène, polypropylène, polytétrafluoroéthylène, polyuréthanes, polyétheruréthanes, polyétheruréthanes de silicone, polyuréthanes de silicone, polycarbonate-uréthanes de silicone, élastomères de polyoléfine, gommes EPDM, fluorosilicones, chitosanes de carboxyméthyl, poly(aryléther éther cétones), poly(éther éther cétones), polyéthylène téréphtalate, polyvalérates, cellulose de carboxyméthyl, cellulose, viscose, triacétates de viscose, nitrates de cellulose, acétates de cellulose, cellulose de hydroxyéthyle, butyrates de cellulose, acetate-butyrates de cellulose, copolymères d'éthyle-vinyle acétate, polysulfones, résines époxy, résines ABS, silicones comme les polysiloxanes, polydiméthyl siloxanes, halogènes de polyvinyle et copolymères, éthers de cellulose, triacétates de cellulose, chitosanes et copolymères et/ ou mélanges de ceux-ci.

33. Procédé selon l'une des revendications 22 - 32, **caractérisé en ce que** l'application de polysaccharides selon la formule I selon la revendication 1 s'effectue par des interactions hydrophobes, forces de van der Waals, interactions electrostatiques, liaison hydrogène, interactions ioniques, réticulation et/ ou liaison covalente.

34. Procédé médical selon l'une des revendications 22 - 33, où au lieu de l'agent actif Paclitaxel un des agents actifs suivants est utilisé : simvastatine, sodium de tialine, sous-oxyde macrocyclique (MCS), dérivés de MCS, protéine C activée (aPC), PETN, trapidil, β-estradiol ainsi que des mélanges de ces agents actifs ou des mélanges d'un de ces agents actifs avec Paclitaxel.

35. Dispositif médical disponible selon un procédé selon l'une des revendications 22 - 34.
